# EUROPEAN PATENT SPECIFICATION

(11) **EP 3 156 795 B1**
(45) Date of publication and mention of the grant of the patent: **29.09.2021**
(21) Application number: 15806578.9
(22) Date of filing: 05.06.2015
(51) Int. Cl.: G16H 10/60, G01N 33/50, G01N 33/48, A61C 19/06, A61B 10/00, G06F 3/023

(54) **TEST RESULT SHEET CREATION DEVICE, TEST RESULT SHEET CREATION METHOD, TEST RESULT SHEET CREATION PROGRAM, TEST RESULT SHEET, AND TESTING APPARATUS**
LABORBEFUNDTABELLENERZEUGUNGSVORRICHTUNG, LABORBEFUNDTABELLENERZEUGUNGSVERFAHREN, LABORBEFUNDTABELLENERZEUGUNGSPROGRAMM, LABORBEFUNDTABELLE UND TESTVORRICHTUNG
DISPOSITIF, PROCÉDÉ ET PROGRAMME DE CRÉATION DE FEUILLE DE RÉSULTAT DE TEST, FEUILLE DE RÉSULTAT DE TEST, ET APPAREIL DE TEST

(30) Priority: 11.06.2014 JP 2014120774
(43) Date of publication of application: 19.04.2017
(73) Proprietor: ARKRAY, Inc., Kyoto-shi, Kyoto 601-8045 (JP)
(72) Inventor: MAKI, Riichi, Tokyo 130-8644 (JP); FUJIKAWA, Haruhiko, Tokyo 130-8644 (JP); SAITO, Kouichi, Tokyo 130-8644 (JP); MIYAKE, Mikio, Tokyo 130-8644 (JP); NISHINAGA, Eiji, Tokyo 130-8644 (JP); SATAKE, Seiji, Kyoto-shi Kyoto 602-0008 (JP); FUKUTA, Isao, Kyoto-shi Kyoto 602-0008 (JP)
(74) Representative: Dehns
(86) International application number: PCT/JP2015/066388
(87) International publication number: WO 2015/190415

(56) References cited:
- EP-A1- 2 660 597
- WO-A1-2012/090995
- JP-A- H08 123 870
- JP-A- 2004 302 523
- JP-A- 2006 081 847
- JP-A- 2007 183 281
- JP-A- 2008 210 171
- JP-A- 2010 277 260
- US-A1- 2002 065 854
- US-A1- 2012 054 230
- None

## Description

### TECHNICAL FIELD

The present invention pertains to an examination result sheet creation apparatus, an examination result sheet creation method, an examination result sheet creation program, an examination result sheet, and an examination apparatus.

### BACKGROUND ART

An intraoral disease risk and an intraoral state instanced by an intraoral hygiene state are required to be known for preventing and curing an intraoral disease. The intraoral disease risk connotes, e.g., a dental caries risk, a periodontal disease and other equivalent risks. The dental caries risk is a risk indicating easiness of morbidity to the dental caries and also the easiness of a progress to the dental caries. The periodontal disease risk is a risk indicating the easiness of morbidity to the periodontal disease and also the easiness of the progress to the periodontal disease. The dental caries is also called a decayed tooth.

The intraoral disease risk and the intraoral hygiene state are diagnosed by measuring individual components, properties and other equivalent elements (examination items) that reflect the intraoral disease risk and the intraoral hygiene state in a way that uses a saliva collected from an examinee (a patient and other equivalent persons) and a mouth wash solution thereof. The examination items for measurement are exemplified by a caries fungus count (e.g., a mutans streptococci count) in the saliva, an acid buffer capacity (neutralizing capacity), an occult blood quantity, and a leukocyte count.

It is known that the caries fungus count in the saliva or the acid buffer capacity of the saliva reflect the caries risk. To be specific, the caries risk is considered higher with a larger caries fungus count or a weaker acid buffer capacity. It is also known that the occult blood quantity and the leukocyte count in the saliva reflect the periodontal disease risk. Specifically, it is known that upon a lesion of gingival tissues concomitant with the periodontal disease, the occult blood is detected in the saliva, and leucocytes concentrate at a morbidity region of the periodontal disease.

Known also is a measurement apparatus (e.g., Patent document 1) configured to display comments based on determined risk levels by measuring parameters that reflect the caries risk, the periodontal disease risk and intraoral cleanness with respect to the saliva obtained from an oral cavity, and determining risk levels of the caries risk, the periodontal disease risk and the intraoral cleanness risk.

### [Documents of Prior Art]

### [Patent Document]

[Patent Document 1] International Publication WO2012/090995, published in English as EP 2660597.

US 2012/054230 discloses an apparatus and method for assisting medical report creation, wherein a sentence may be created on the basis of words and clauses which are selectable from multiple options, as well as for providing doctors with medical information. Further disclosed is means to assign a diagnosis on the basis of the examination results input into the system.

### SUMMARY OF THE INVENTION

### [Problems to be solved by the Invention]

In the measurement apparatus described in the Patent document 1, the user (the dentist) displays the measurement results of the examination items pertaining to the intraoral disease risk and the intraoral hygiene state to the examinee (the patient), in which case a single comment predetermined based on the measurement result per examination item is displayed together with the measurement results.

However, with such a single comment for the saliva examination, when the user (the dentist) performs other examinations like examinations about an intraoral diagnosis result and a lifestyle of the examinee (the patient) in addition to the saliva examination, results of these examinations are not reflected as the case may be.

On the other hand, when the user (the dentist) performs other examinations in addition to the result of the saliva examination, and comments based on the measurement results of the saliva examination are set freely describable, in which case it is difficult to provide unified comments, resulting in such a problem that there occurs a dispersion in evaluation of the intraoral state irrespective of obtaining the similar examination results.

It is an object of the present invention to provide a technology capable of providing measurement results of a saliva examination together with more proper comments.

### [Means for solving the Problems]

A technology of the disclosure adopts the following means in order to solve the problems described above.

According to a first aspect, the invention provides a system comprising an examination result sheet creation apparatus and an external apparatus, the examination result sheet creation apparatus comprising: a display unit configured to display examination results of a saliva examination; a control unit configured to create an examination result sheet containing at least one examination result and a comment corresponding to the at least one examination result; a storage unit configured to store data used by the examination result sheet creation apparatus; and a communication unit configured to output information of the at least one examination result and a comment to the external apparatus, characterized in that: the display unit is configured to display a plurality of comment candidates corresponding to the at least one examination result; and the examination result sheet creation apparatus comprises an input unit configured to accept an input of a selection result of the plurality of comment candidates and configured to accept an input of a free descriptive comment, wherein the control unit is further configured to: cause the display unit to display an input field of a free descriptive comment about the examination result of the saliva examination, the free descriptive comment being inputted to the input field by using the input unit; accept the free descriptive comment inputted to the input field; store the at least one examination result and a comment selected from the comment candidates or the comment inputted as the free descriptive comment as being associated with each other in a comment database on the storage unit; and store the free descriptive comment, if inputted, as one of the plurality of comment candidates corresponding to the at least one examination result on the storage unit; wherein the comment in the examination result sheet comprises the comment selected from the comment candidates or the free descriptive comment, and wherein the comment output by the communication unit is the associated comment selected from the comment candidates or the associated comment inputted as the free descriptive comment to the external apparatus, and wherein the external apparatus is configured to: accumulate the comments associated with each examination result; determine a comment usage frequency for each examination result; generate a new comment database on the basis of the comment usage frequency; and distribute the new comment database to the examination result sheet creation apparatus.

According to a second aspect, the present invention provides an examination result sheet creation method by which an examination result sheet creation apparatus executes: displaying examination results of a saliva examination and a plurality of comment candidates corresponding to the examination results and an input field of a free descriptive comment about the examination result; accepting an input of a selection result of the plurality of comment candidates or the free descriptive comment inputted to the input field; storing at least one examination result and the comment selected from the comment candidates or the free descriptive comment inputted to the input field by being associated with each other in a comment database on a storage unit, storing the free descriptive comment, if inputted to the input field, as one of the plurality of comment candidates corresponding to the examination results on the storage unit, creating an examination result sheet containing at least one examination result and the comment selected from the comment candidates or the free descriptive comment inputted to the input field; and outputting information of the at least one examination result and the associated comment selected from the comment candidates or the associated free descriptive comment inputted to the input field, to an external apparatus, and by which the external apparatus executes: accumulating the comments associated with each examination result; determining a comment usage frequency for each examination result; generating a new comment database on the basis of the comment usage frequency; and distributing the new comment database to the examination result sheet creation apparatus.

### [Effect of the Invention]

According to the present invention, it is feasible to provide the technology capable of providing the measurement results of the saliva examination together with more proper comments.

### BRIEF DESCRIPTION OF THE DRAWINGS

FIG. 1 is a diagram illustrating an example of a configuration of an examination result sheet creation system.
FIG. 2 is a diagram illustrating an example of a configuration of an examination result sheet creation apparatus.
FIG. 3 is an example of a hardware configuration of an information processing apparatus.
FIG. 4 is a flowchart illustrating an example (1/2) of an operation flow of creating an examination result sheet by the examination result sheet creation apparatus in an embodiment 1.
FIG. 5 is a flowchart illustrating an example (2/2) of the operation flow of creating the examination result sheet by the examination result sheet creation apparatus in the embodiment 1.
FIG. 6 is a diagram illustrating an example of a patient information input screen.
FIG. 7 is a diagram illustrating an example of a dental record table.
FIG. 8 is a diagram illustrating an example of the patient information input screen after a patient name and other equivalent items have been reflected.
FIG. 9 is a diagram illustrating an example of the dental record table after receiving measurement results.
FIG. 10 is a diagram illustrating an example of a measurement result screen.
FIG. 11 is a diagram illustrating an example of comments about an examination item "occult blood quantity" and a rank "small" in a comment DB.
FIG. 12 is a diagram illustrating an example of the comments about the examination item "occult blood quantity" and a rank "intermediate" in the comment DB.
FIG. 13 is a diagram illustrating an example of the comments about the examination item "occult blood quantity" and a rank "numerous" in the comment DB.
FIG. 14 is a diagram illustrating an example of a comment determination screen.
FIG. 15 is a diagram illustrating an example of a comment determination screen when selecting a comment field of "occult blood quantity" on the comment determination screen in FIG. 14.
FIG. 16 is a diagram illustrating an example of a clinic name/dentist name input screen.
FIG. 17 is a diagram illustrating an example of an examination result sheet created by the examination result sheet creation apparatus.
FIG. 18 is a diagram illustrating an example of a clinic name table.
FIG. 19 is a diagram illustrating an example of a dentist name table.
FIG. 20 is a diagram illustrating an example of the clinic name/dentist name input screen in a modified example 1 of the embodiment 1.
FIG. 21 is a flowchart illustrating an example (1/2) of an operation flow of creating the examination result sheet by the examination result sheet creation apparatus in an embodiment 2.
FIG. 22 is a flowchart illustrating an example (2/2) of the operation flow of creating the examination result sheet by the examination result sheet creation apparatus in the embodiment 2.
FIG. 23 is a diagram illustrating an example of an intraoral state determination screen.
FIG. 24 is a diagram illustrating an example of a contribution table.
FIG. 25 is a diagram illustrating an example of a comment table with respect to an item "toothbrushing time" of a dental care method, a contribution "large" or "intermediate" in a dental care comment DB.
FIG. 26 is a diagram illustrating an example of a comment table with respect to an item "toothbrushing time" of a dental care method, a contribution "small" in the dental care comment DB.
FIG. 27 is a diagram illustrating an example of the comment table with respect to "product A" as a dental care product and a contribution "large" or "small" in the dental care DB.
FIG. 28 is a diagram illustrating an example of a dental care comment determination screen.
FIG. 29 is a diagram illustrating an example of the dental care comment determination screen when selecting a comment field of "toothbrushing time" on the dental care comment determination screen in FIG. 28.
FIG. 30 is a diagram illustrating an example (1/2) of the examination result sheet created by the examination result sheet creation apparatus.
FIG. 31 is a diagram illustrating an example (2/2) of the examination result sheet created by the examination result sheet creation apparatus.

### EMBODIMENTS

### [Mode for Carrying out the Invention]

Embodiments will hereinafter be described with reference to the drawings.

### [Embodiment 1]

### (Example of Configuration)

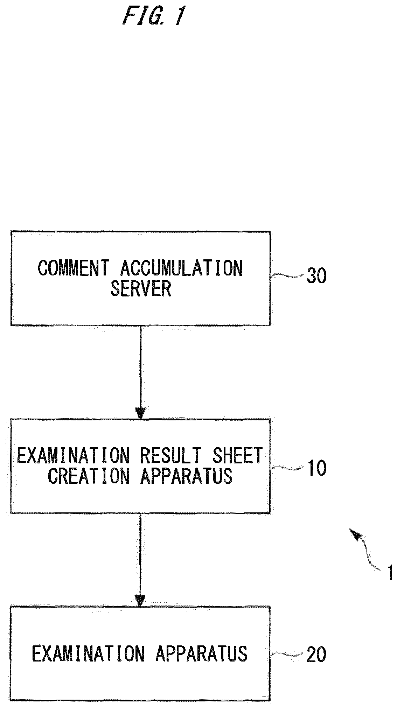

FIG. 1 is a diagram illustrating an example of a configuration of an examination result sheet creation system in an embodiment 1. An examination result sheet creation system 1 in FIG. 1 includes an examination result sheet creation apparatus 10, an examination apparatus 20, and a comment accumulation server 30. The examination result sheet creation apparatus 10 creates an examination result sheet based on a result of an examination made by the examination apparatus 20. The examination apparatus 20 measures predetermined examination items with respect to saliva (examination object) of an examinee (a patient and other equivalent persons), and notifies the examination result sheet creation apparatus 10 of measured results. The comment accumulation server 30 accumulates comments used on the occasion of creating an examination result sheet in the examination result sheet creation apparatus 10. The comment accumulation server 30 and the examination result sheet creation apparatus 10 are interconnected via, e.g., a network. A plurality of examination result sheet creation apparatuses 10 can be connected to the comment accumulation server 30.

In the examination result sheet creation system 1 according to embodiment 1, the examination apparatus 20 measures the predetermined examination items by using the saliva of the examinee (the patient). The examination result sheet creation apparatus 10 presents, to a dental doctor and other equivalent experts, comment selective options per examination item, based on measurement results of the examination by the examination apparatus 20, and creates the examination result sheet containing the comment selected or inputted by the dental doctor and the measurement results.

Herein, the predetermined examination items about the saliva of the examinee (the patient) are measured beforehand, and the measurement results are previously stored in a storage unit 12, whereby the examination result sheet creation apparatus 10 may create the examination result sheet. The examination result sheet creation apparatus 10 may also create the examination result sheet by acquiring the measurement results of the examination about the saliva of the examinee (the patient) via the network and other equivalent communication links).

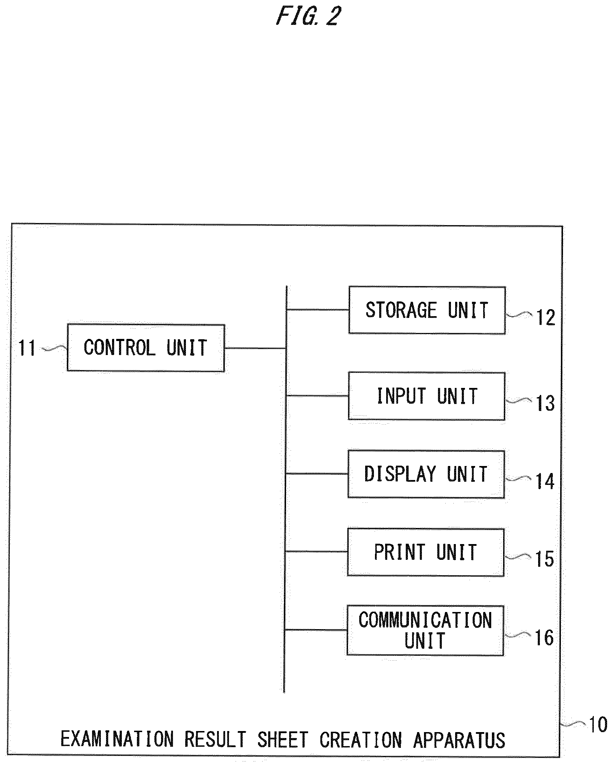

FIG. 2 is a diagram illustrating an example of a configuration of the examination result sheet creation apparatus. The examination result sheet creation apparatus 10 in FIG. 2 includes a control unit 11, the storage unit 12, an input unit 13, a display unit 14, a print unit 15 and a communication unit 16.

The control unit 11 controls whole operations of the examination result sheet creation apparatus 10. The control unit 11 may also control the operation of the examination apparatus 20 via the communication unit 16.

The storage unit 12 stores data used by the examination result sheet creation apparatus 10, dental record (Karte) tables containing information of the examinees (the patients), and data of a comment database (DB) containing the comments corresponding to the examination items and the measurement results.

The input unit 13 accepts an input of characters and other equivalent codes by a user (a dentist and other equivalent practitioners), and selection and manipulation of a button and other equivalent input elements. The input unit 13 is attained by a keyboard, a pointing device and other equivalent devices.

The display unit 14 displays the information of the examinee (the patient), the measurement results, the comments for the measurement results, care guidances or comments pertaining to dental care products, and instructions for the user (the dentist). The display unit 14 is attained by, e.g., a display device like a display and other equivalent devices.

The print unit 15 prints, on printing paper, the examination result sheet containing the measurement results and the comments about the measurement results on the basis of an instruction given from the control unit 11. The print unit 15 is attained by a printing device instanced by a printer.

The communication unit 16 performs communications of the data with other apparatuses, i.e., the examination apparatus 20, the comment accumulation server 30 and other equivalent apparatuses. External devices instanced by an external storage device can be connected to the communication unit 16.

The examination result sheet creation apparatus 10 enables the user (the dentist) to harvest and measure the saliva by employing the examination apparatus 20 such as displaying an operation method of the examination apparatus 20. The examination apparatus 20, upon finishing the examination of the saliva, transmits the measurement results of examining the saliva to the examination result sheet creation apparatus 10. The examination result sheet creation apparatus 10 receives the measurement results of the examination from the examination apparatus 20. The received measurement results of the examination are stored in the dental record table per examinee (patient).

The examination result sheet creation apparatus 10 may previously measure the predetermined examination items about the saliva of the examinee (the patient), and may acquire the measurement results of the examination that are stored in the storage unit 12. The examination result sheet creation apparatus 10 may also acquire the measurement results of the examination about the saliva of the examinee (the patient) via the network.

For example, the examination apparatus 20 measures the predetermined examination items by dispensing a drop of a 3 ml mouth wash solution, into which the examinee (the patient) washes a mouth by distilled water for 10 sec, on test paper, and transmits the measurement results to the examination result sheet creation apparatus 10. Herein, the predetermined examination items are seven items, i.e., a caries fungus count of the saliva, pH, an acid buffer capacity, an occult blood quantity, a leukocyte count, a protein concentration, and an ammonia concentration. A reagent corresponding to each examination item is applied onto the test paper. The examination apparatus 20 measures the caries fungus count as the examination item by detecting a color in color reaction between the reagent and the saliva of the examinee (the patient) on the test paper. A measurement method is not limited to the method described herein.

Herein, the caries fungus count, the pH and the acid buffer capacity are used as parameters reflecting a dental caries risk. The parameters reflecting the dental caries risk are not limited to those described above, but may involve using glucosyltransferase activity, a sucrose level, a glucose level, an organic acid concentration, a lactate concentration, a calcium concentration, a phosphate concentration, and a reactivity to a mutans streptococci immune body.

The caries fungi (mutans streptococci) are pathogenic bacteria of the dental caries. The dental caries risk is considered higher with a larger number of caries fungi. The pH of the saliva decreases due to acid produced by the caries fungi. When a large quantity of acid is produced, enamel on a tooth surface is dissolved, with the result that the dental caries get progressed. The dental caries risk is considered higher with a lower level of the pH of the saliva. The acid buffer capacity indicates resistance against the acid produced by the caries fungi. Hence, the dental caries risk is considered higher with a lower acid buffer capacity.

Herein, the occult blood quantity, the leukocyte count and the protein concentration are used as the parameters reflecting a periodontal disease risk. The parameters reflecting the periodontal disease risk are not limited to those described above, but may involve using a calcium concentration, an alkaline phosphatase activity, a nitrite concentration, a lactate dehydrogenase activity, a lipopolysaccharide concentration, a reactivity to a periodontal disease immune body, a γ-GTP concentration, an albumin concentration, an antioxidant degree, an IgA concentration, a cystatin concentration, and an a1-antitrypsin concentration.

The occult blood quantity in the saliva is detected upon a lesion of gingival tissues concomitant with the periodontal disease. A lesion degree of periodontal tissues can be therefore known by measuring the occult blood quantity. It is considered that the lesion degree of the periodontal tissues becomes higher and the periodontal disease risk becomes higher with the larger occult blood quantity. The leukocytes are to be concentrated on a morbidity region of the periodontal disease. Hence, an inflammatory degree of the periodontal tissues can be known by measuring the leukocyte count. It is considered that the inflammatory degree of the periodontal disease becomes higher and the periodontal disease risk becomes higher with a larger leukocyte count. It is also considered that a fungus count of the periodontal disease becomes larger and the periodontal disease risk becomes higher with a higher protein concentration in the saliva.

Herein, the ammonia concentration is used as the parameter reflecting intraoral cleanness. The parameter reflecting the intraoral cleanness is not limited to the ammonia concentration, but may involve using the protein concentration, a total fungus count, a turbidity, a viscosity and a secrete quantity.

It is known that the intraoral fungi are actively propagated, in which case the ammonia concentration in the saliva gets high. The intraoral cleanness is therefore considered to be lower as the ammonia concentration becomes higher.

The comment accumulation server 30 accumulates the comments used when creating the examination result sheet in the examination result sheet creation apparatus 10. The comment accumulation server 30 accumulates the comments used when creating the examination result sheet, which are transmitted from the examination result sheet creation apparatus 10, and extracts the frequently used comments.

The examination result sheet creation apparatus 10 can be attained by employing a dedicated or general-purpose computer (information processing apparatus) instanced by a Personal Computer (PC), a workstation (WS), a Personal Digital Assistant (PDA), or by employing an electronic equipment mounted with the computer. The examination result sheet creation apparatus 10 can be attained by employing a smartphone, a mobile phone, a tablet terminal, the dedicated or general-purpose computer, or the electronic equipment mounted with the computer. The comment accumulation server 30 can be attained by employing the dedicated or general-purpose computer (information processing apparatus) instanced by the Personal Computer (PC), the workstation (WS), the Personal Digital Assistant (PDA), or by employing the electronic equipment mounted with the computer.

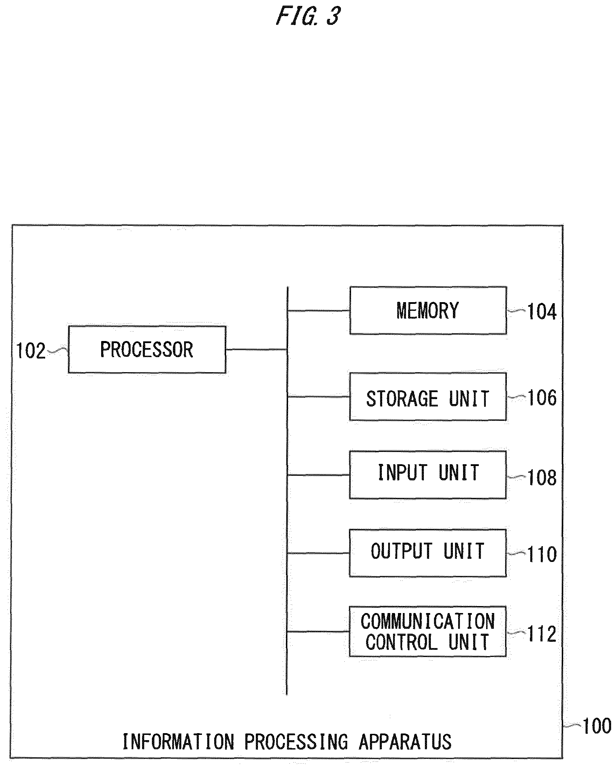

FIG. 3 is a diagram illustrating an example of a hardware configuration of an information processing apparatus. The examination result sheet creation apparatus 10 is attained by an information processing apparatus 100 as depicted in FIG. 3. The information processing apparatus 100 includes a processor 102, a memory 104, a storage unit 106, an input unit 108, an output unit 110, and a communication unit 112. The memory 104 and the storage unit 106 are non-transitory computer readable recording mediums. The hardware configuration of the information processing apparatus is not limited to the example illustrated in FIG. 3, and the components thereof may be properly omitted, replaced and added.

The processor 102 loads programs stored on the recording medium onto a work area of the memory 104 and runs the programs, and the respective constructive units are controlled through running the programs, whereby the information processing apparatus 100 can attain functions conforming to predetermined purposes.

The processor 102 is exemplified by a Central Processing Unit (CPU) and a Digital Signal Processor (DSP).

The memory 104 includes a Random Access Memory (RAM) and a Read Only Memory (ROM). The memory 104 is also called a main storage device.

The storage unit 106 is exemplified by an Erasable Programmable ROM (EPROM) and a Hard Disk Drive (HDD). The storage unit 106 can include a removable medium, i.e., a portable recording medium. The removable medium is a disc recording medium instanced by a Universal Serial Bus (USB) memory or a Compact Disc (CD) and a Digital Versatile Disc (DVD). The storage unit 106 is also called a secondary storage device.

The storage unit 106 stores various categories of programs, various items of data and various types of tables in a readable/writable manner on the recording medium. An Operating System (OS), the various categories of programs and the various types of tables are stored on the storage unit 106. Information stored on the storage unit 106 may also be stored on the memory 104. Information stored on the memory 104 may also be stored on the storage unit 106.

The OS is software that functions as an intermediary between software and hardware, and performs managements of a memory space, files, processes and tasks. The OS includes a communication interface. The communication interface is a program that transfers and receives the data to and from other external apparatuses interconnected via a communication control unit 112. The external apparatuses include, e.g., other information processing apparatuses, external storage devices and other equivalent devices.

The input unit 108 includes a keyboard, a pointing device, a wireless remote controller, a touch panel, and other equivalent devices. The input unit 108 can include a video/still image input device like a camera, and a voice input device like a microphone.

The output unit 110 includes a display device instanced by a Cathode Ray Tube (CRT) display, a Liquid Crystal Display (LCD), a Plasma Display Panel (PDP) and an Electroluminescence (EL) panel, and an output device instanced by a printer. The output unit 110 can include a voice output device like a loudspeaker.

The communication control unit 112 establishes connections with other apparatuses, and thus controls the communications between the information processing apparatus 100 and other apparatuses. The communication control unit 112 is exemplified by a Universal Serial Bus (USB) interface board, a video output circuit, a Local Area Network (LAN) interface board, a wireless communication circuit for wireless communications, and a communication circuit for telephone communications. The LAN interface board and the wireless communication circuit are connected to a network instanced by the Internet. The communication circuit for the telephone communications is connected to a telephone communication network. For example, the information processing apparatus 100 is connected to the examination apparatus 20 via the communication control unit 112.

The input unit 108 and the output unit 110 may be an input device and an output device respectively as peripheral devices independent of the information processing apparatus 100. Hereat, the input device and the output device are connected to the information processing apparatus 100 via the communication control unit 112 of the information processing apparatus 100.

The processor loads the programs stored on the secondary storage device onto the main storage device and runs the programs, whereby the information processing apparatus to attain the examination result sheet creation apparatus 10 implements functions as the control unit 11, the input unit 13, the display unit 14, the print unit 15 and the communication unit 16. On the other hand, the storage unit 12 is provided in a storage area of the main storage device or the secondary storage device.

The respective units of the examination result sheet creation apparatus 10 can be respectively attained as hardware components, software components or combinations thereof.

The hardware components are hardware circuits instanced by a Field Programmable Gate Array (FPGA), an Application Specific Integrated Circuit (ASIC), a gate array, a combination of logic gates, and an analog circuit.

The software components are components for implementing predetermined processes softwarewise. The software components are not concepts that limit programming languages to attain the software, and development environments.

Steps of describing the program include processes that are, as a matter of course, executed in time-series along a described sequence, and processes that are not necessarily executed in time-series but are executed in parallel or individually. Part of the steps describing the program may be omitted.

### (Operational Example)

Described is an operational example of how the examination result sheet creation apparatus 10 creates the examination result sheet. Given herein is the operational example of the examination result sheet creation apparatus 10 when the user (the dentist)) of the examination result sheet creation apparatus 10 creates the examination result sheet by examining the saliva (examination) of the examinee (the patient) by employing the examination apparatus 20.

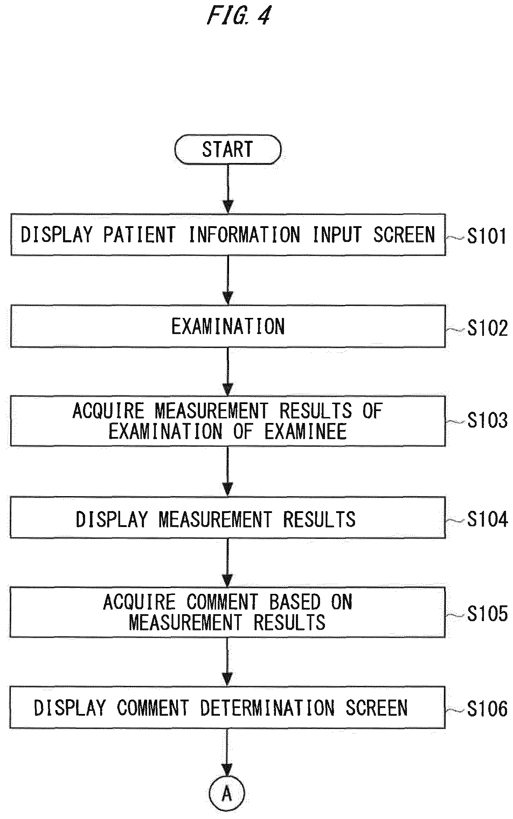

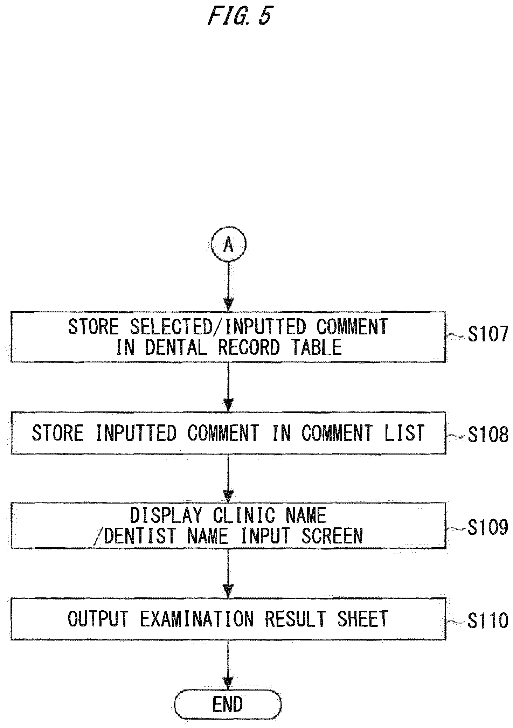

FIGS. 4 and 5 are diagrams each illustrating an example of an operation flow along which the examination result sheet creation apparatus 10 creates the examination result sheet. A symbol "A" in FIG. 4 is continued to "A" in FIG. 5. The examination result sheet creation apparatus 10 is employed by the user, i.e., the dentist.

In step S101, the control unit 11 of the examination result sheet creation apparatus 10 displays a patient information input screen for inputting information of the examinee (the patient) on the display unit 14. The control unit 11 waits for the user (the dentist) to input the information of the examinee (the patient).

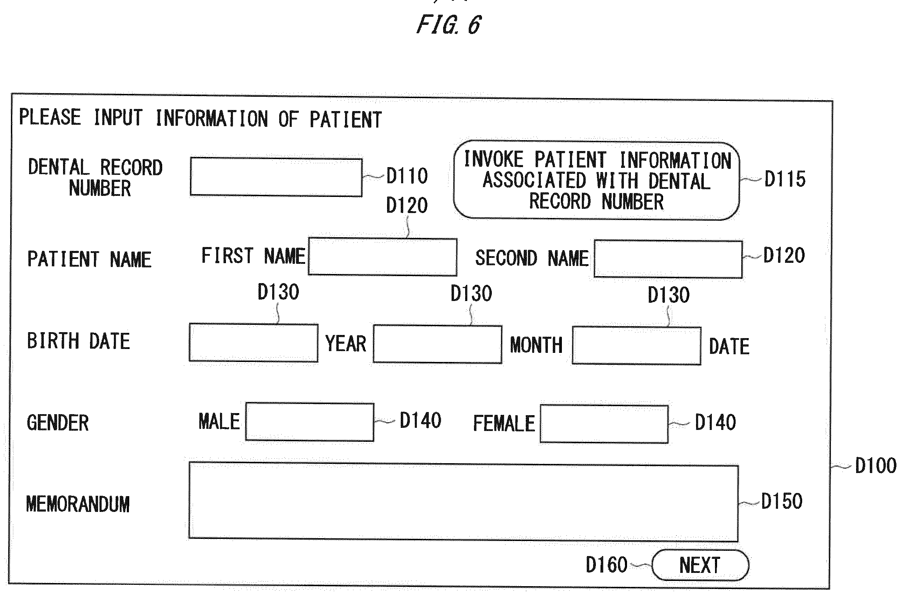

FIG. 6 is a diagram illustrating an example of the patient information input screen. A patient information input screen D100 contains a dental record number input field D110, a patient name input field D120, a birth date input field D130, a gender input field D140, and a memorandum input field D150. The patient information input screen D100 further contains a "dental record number based patient information call" button D115, and a "next" button D160. The user (the dentist) inputs respective items of information of the examinee (the patient) to the respective input fields via the input unit 13.

When an examinee (a patient) to undergo an examination from now is the examinee (the patient) having undergone the examination in the past, the dental record table pertaining to the examinee (the patient) concerned exists in the storage unit 12. The dental record table is a table for storing the information of the examinee (the patient). One dental record table is created for every one examinee (the patient) and is identified by a dental record number. Hereat, the user (the dentist) inputs the dental record number to the dental record number input field D110 on the patient information input screen D100, and, when selecting the "dental record number based patient information call" button D115, the control unit 11 acquires the dental record table associated with the inputted dental record number from the storage unit 12. A variety of buttons within the screen displayed on the display unit 14 are selected by an input manipulated on the keyboard serving as the input unit 13, and a click manipulated on the pointing device as the input unit 13.

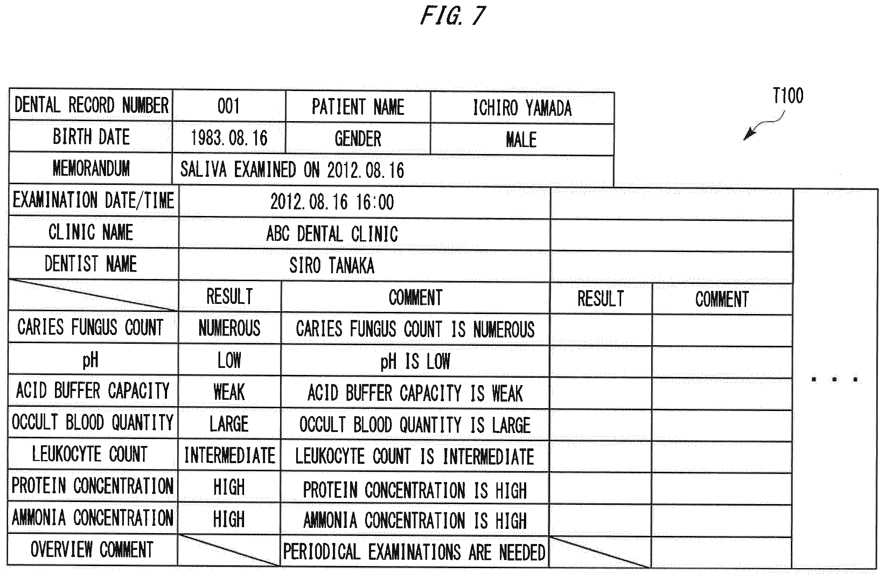

FIG. 7 is a diagram illustrating an example of the dental record table. A dental record table T100 is stored on the storage unit 12. The information of the examinee (the patient) is stored in the dental record table T100 per examinee (per patient). Each dental record table T100 is identified by the dental record number. In other words, the examinee (the patient) is identified by the dental record number. The dental record table T100 contains the dental record number, a name of the patient, the birth date, the gender and the memorandum. The dental record table can contain results of all examinations pertaining to one examinee (the patient) identified by the dental record number. The dental record number may contain characters other than numerals. Results of the examination contain a date/time of the examination, a name of a dental clinic and a doctor name of the dentist per examination. The name of the dental clinic and the doctor name of the dentist are respectively the name of the dental clinic and the doctor name of the dentist taking a charge of the examination. The results of the examination contain the measurement results of the examination items such as the caries fungus count, the pH, the acid buffer capacity, the occult blood quantity, the leukocyte count, the protein concentration and the ammonia concentration, and comments made by the user (the dentist) with respect to the measurement results of the respective examination items per examination. The results of the examination further contain an overview comment made by the user (the dentist) per examination. The dental record table T100 in FIG. 7 stores the dental record number, the information of the examinee's name (the patient's name), the measurement results of the past examinations (examination date/time: 2012.08.16 16:00), the comments about the measurement results, the overview comment, the name (clinic name) of the dental clinic taking charge of the examination, and the name of the dentist (dentist name) taking charge of the examination. The dental record table T100 may contain other items pertaining to the examinee (the patient).

The dental record table T100 may directly store actual values (measurement values) as the measurement results of the respective examination items, and may also store ranks such as "high (numerous, strong)", "intermediate" and "low (very few, weak)" by classifying the actual values into a plurality of ranks, i.e., "high (numerous, strong)", "intermediate" and "low (very few, weak)" on the basis of a predetermined threshold value. The classification can provide a rank "high (numerous, strong)" with the actual value being equal to or larger than a first threshold value, a rank "intermediate" with the actual value being smaller than the first threshold value but equal to or larger than a second threshold value, and a rank "low (very few, weak)" with the actual value being smaller than the second threshold value. Herein, the first threshold value is larger than the second threshold value. Each threshold value is determined per examination item. The dental record table T100 may store both of the actual values and the ranks. In the example in FIG. 7, the ranks are stored as the measurement results of the respective examination items.

Herein, through classified into the ranks at three stages such as "high (numerous, strong)", "intermediate" and "low (very few, weak)", a number of stages of the classification is not limited to "3". The number of stages of the classification may be equal to or larger than "2", and may also be increased or decreased depending on the examination items. Classification in the below-mentioned comment DB is also varied corresponding to the number of stages of the classification.

The control unit 11, when acquiring the dental record table T100 from the storage unit 12, extracts the name of the patient, the birth date, the gender and the memorandum from the dental record table T100. The control unit 11 displays the patient information input screen D100 on the display unit 14 in a way that reflects the extracted name of the patient and other items in the corresponding input fields.

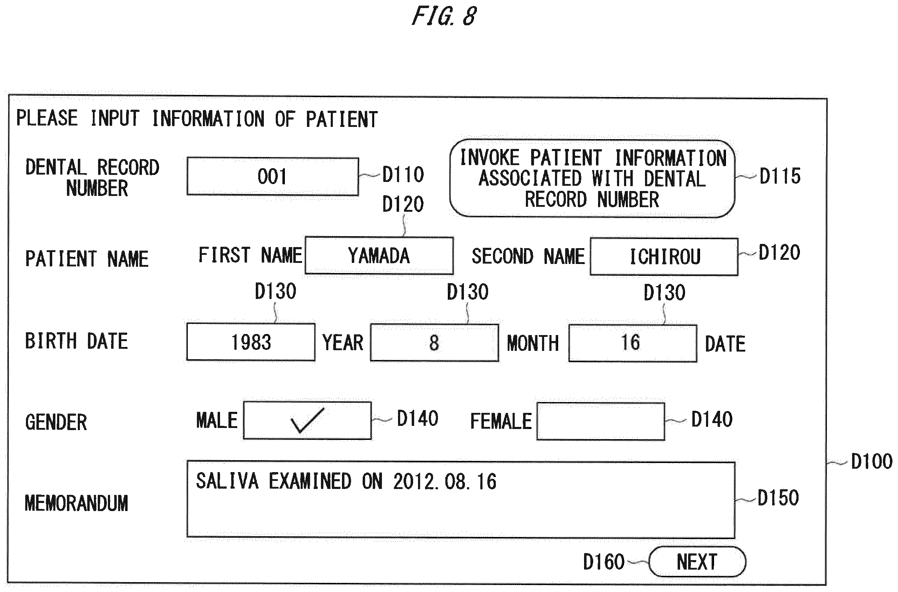

FIG. 8 is a diagram illustrating an example of the patient information input screen after the name of the patient and other equivalent items have been reflected in the input fields. In the example of FIG. 8, pieces of information such as the name of the patient and other equivalent items are reflected in the respective input fields on the basis of the dental record table T100.

The user (the dentist) checks the information of the examinee (the patient) displayed on the display unit 14, then makes a change when there is an item to be changed, and selects the "next" button. The control unit 11, upon selecting the "next" button, checks whether the information of the examinee (the patient) is changed or not, then, when the changed item exists, updates the dental record table T100 with respect to the changed item, and stores the updated dental record table T100 on the storage unit 12.

When the examinee (the patient) undergoing the examination from now is an examinee (a patient) not undergoing any examinations in the past, any dental record table pertaining to this examinee (the patient) does not exist in the storage unit 12. Accordingly, the user (the dentist) inputs the name, the birth date, the gender and the memorandum of the examinee (the patient) to the respective input fields together with the dental record number. The user (the dentist), upon inputting the information of the examinee (the patient), selects the"next" button. The control unit 11, when the"next" button D160 is selected, creates a new dental record table T100 in the storage unit 12, and stores the examinee (the patient) information inputted to the respective input fields in the created dental record table T100.

After selecting the "next" button D160 and when updating or creating the dental record table T100, the processing advances to step S102.

In step S102, the control unit 11 of the examination result sheet creation apparatus 10 displays an examination procedure and other equivalent matters on the display unit 14, causes the examination apparatus 20 to measure the examination items about the saliva of the examinee (the patient) via the communication unit 16.

The examination result sheet creation apparatus 10 displays the examination procedure of examining the saliva on the display unit 14. Based on the examination procedure displayed on the display unit 14, the user (the dentist), after harvesting the saliva from the examinee (the patient) whose information is inputted in step S101, transmits a signal indicating a start of the measurement to the examination result sheet creation apparatus 10 from the examination apparatus 20. The examination result sheet creation apparatus 10 displays a measurement procedure and other equivalent matters on the display unit 14. The user (the dentist) sets the test paper on which the saliva is harvested in the examination apparatus 20 on the basis of the measurement procedure displayed on the display unit 14. The examination result sheet creation apparatus 10 causes the examination apparatus 20 to measure the predetermined examination items with respect to the thus-set test paper. Upon finishing measuring the predetermined examination items, the examination apparatus 20 transmits a signal indicating an end of the measurement to the examination result sheet creation apparatus 10.

In step S103, the control unit 11 of the examination result sheet creation apparatus 10, when receiving the signal indicating the end of the measurement via the communication unit 16 from the examination apparatus 20, instructs the examination apparatus 20 to transmit the examination date/time and the measurement results. The examination apparatus 20 transmits the examination date/time and the measurement results to the examination result sheet creation apparatus 10. The examination apparatus 20 may also transmit the examination date/time and the measurement results together with the signal indicating the end of the measurement to the examination result sheet creation apparatus 10.

The examination result sheet creation apparatus 10 stores the examination date/time and the measurement results acquired from the examination apparatus 20 in the dental record table T100 of the examinee (the patient) undergoing the examination. The examination date/time is stored together with the measurement results in the dental record table T100.

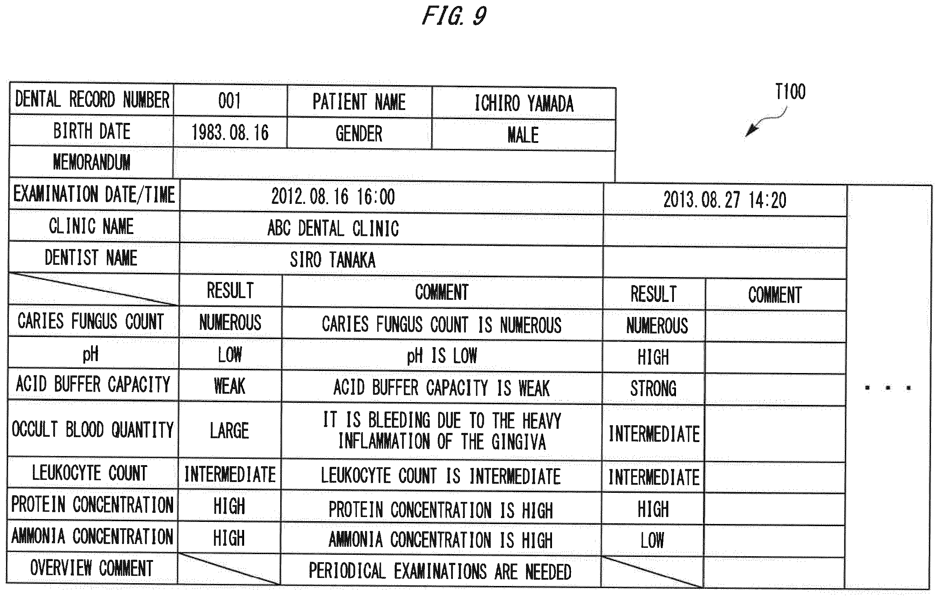

FIG. 9 is a diagram illustrating the dental record table after receiving the measurement results. In the example of FIG. 9, the measurement results of the examination (the examination date/time: 2013.08.27 14:20 of this time are stored in the dental record table T100. In the example of FIG. 9, e.g., the items of a "decayed tooth fungus count being "numerous" and the "pH" being "high" and other items are stored as the measurement results of the examination of this time. In the examination of this time, the comment, the name of the dental clinic and the name of the dentist are not yet inputted, and hence null values are given to the comment field, the dental clinic name field and the dentist field.

In step S104, the control unit 11 of the examination result sheet creation apparatus 10 displays a measurement result screen of the examination performed this time in step S102 on the display unit 14.

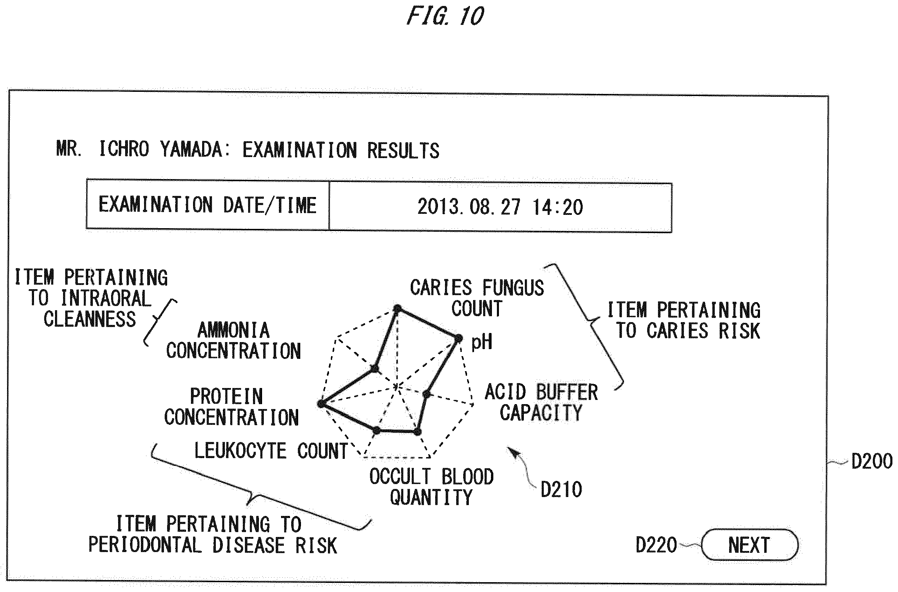

FIG. 10 is a diagram illustrating an example of the measurement result screen. The name of the examinee (the patient), the examination date/time, a measurement result chart D210 and a "next" button D220 are displayed on a measurement result screen D200 in FIG. 10. The measurement results are displayed in the form of the chart D210 indicating high/low levels of the measurement results of the respective examination items in FIG. 10.

The chart D210 takes a heptagon having seven angles matching to the number of the examination items. Seven axes extending toward respective apexes from a center of the heptagon correspond to the examination items, respectively. Positions on the axes extending toward the respective apexes from the center of the heptagon correspond to degrees (ranks) of the examination items. Herein, the position closer to the center on the axis corresponds to a better value of the examination item. In FIG. 10, points on the respective axes in the chart D210 represent the measurement results of the examination items. As in FIG. 10, a chart (a radar chart) indicating the measurement results of the examination items is depicted by connecting the points on the adjacent axes by straight lines.

The control unit 11 of the examination result sheet creation apparatus 10 may display the measurement results of the past examinations together with the measurement results of the examination of this time on the measurement result screen D200. The user (the dentist) can visually recognize the measurement results through the measurement result screen D200. When the user (the dentist) selects the "next" button D220, the processing advances to step S105.

In step S105, the control unit 11 acquires, based on the measurement results, the comments from the comment DB of the storage unit 12 per examination item. The comment DB is previously stored on the storage unit 12. The comment DB contains a comment table about all the examination items and ranks. Each comment table contains the plurality of comments. The comment is a message provided to the examinee (the patient) together with the measurement results.

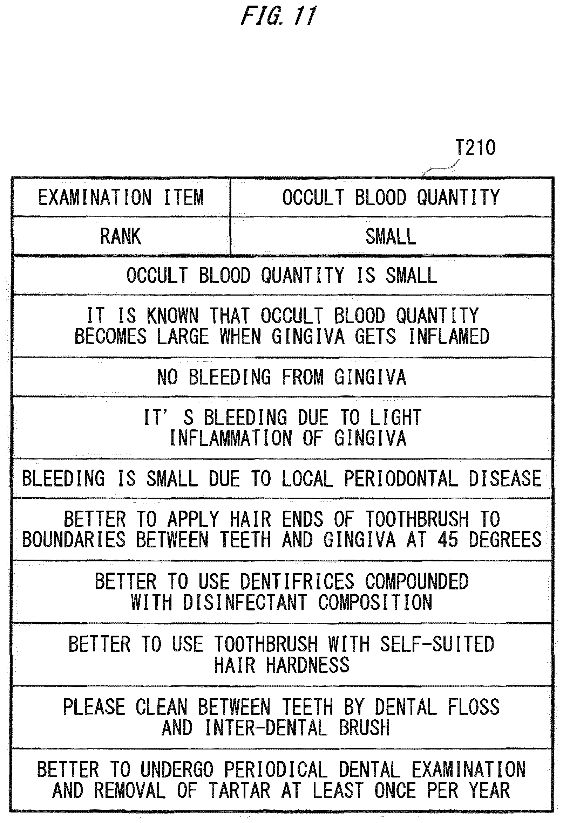

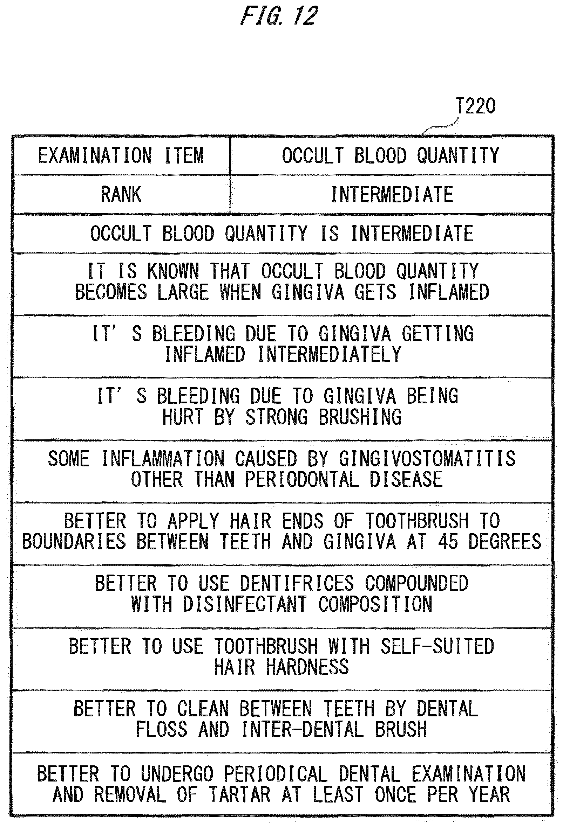

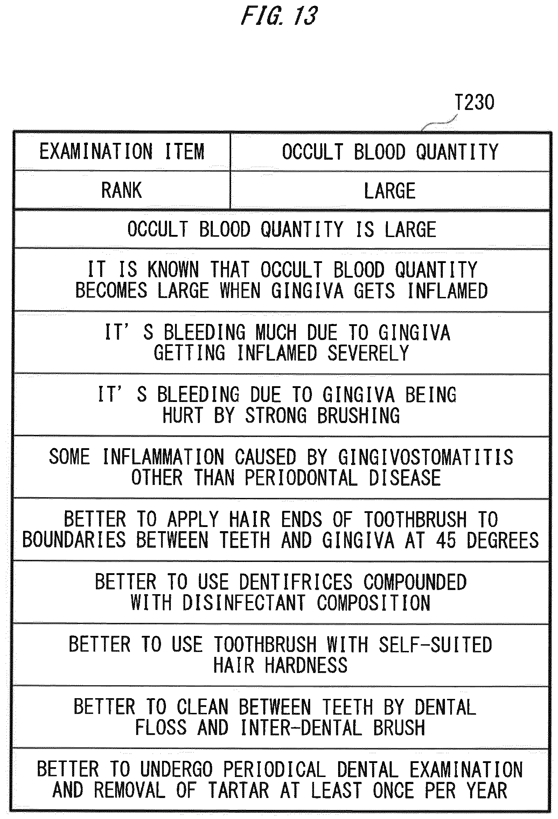

FIGS. 11, 12 and 13 are diagrams illustrating examples of the comments with respect to the examination item "occult blood quantity" in the comment DB. FIG. 11 illustrates a comment table T210 indicating the examination item being the "occult blood quantity" and the rank being "low" in the comment DB. FIG. 12 illustrates a comment table T220 indicating the examination item being the "occult blood quantity" and the rank being "intermediate" in the comment DB. FIG. 13 illustrates a comment table T230 indicating the examination item being the "occult blood quantity" and the rank being "numerous" in the comment DB. Each comment table contains the plurality of comments.

In the example of FIG. 11, the comment table T210 contains five types of comments as the comments about the examination item being the "occult blood quantity" and the rank being "low", such as "the occult blood quantity is small", and "it is known that the occult blood quantity becomes large when the gingiva gets inflamed". These comments are the comments considered proper for being provided to the examinee (the patient) when the measurement result of the 'occult blood quantity" is low. Each comment of the predetermined rank in the predetermined examination item may not, however, be proper for all the examinees (the patients), and it may be sufficient that the comment is proper for at least some examinees. Each comment table contains the comments that can be assumed with respect to the examination items and the ranks. The comments may include those having a low possibility of being assumed. The comments further include a fact based comment such as "the occult blood quantity is small", a known relation based comment between the intraoral condition and the examination item such as "it is known that the occult blood quantity becomes large when the gingiva gets inflamed", and a diagnosis related comment such as "it's bleeding due to the light inflammation of the gingiva". The comments may also include a care method related comment such as "please apply hair ends of a toothbrush to boundaries between the teeth and the gingiva at an angle of 45 degrees", and a dental care product related comment such as "please use dentifrices compounded with a disinfectant composition". The comments can help the user (the dentist) assume every possibility about the intraoral condition of the examinee (the patient).

In the case of the examinee (the patient) undergoing the examination in step S102, for example, the rank is "intermediate" about the examination item "occult blood quantity" in the examination of this time, and hence the control unit 11 acquires the comments contained in the comment table T220. The control unit 11 acquires, based on the ranks about other examination items, the comments contained in the comment table of the comment DB.

In step S106, the examination result sheet creation apparatus 10 displays a comment determination screen on the display unit 14. The comment determination screen is a screen containing the measurement result, a comment field, and an overview comment input field per examination item. The user (the dentist) determines the comment with respect to each examination item, and inputs the overview comment on the comment determination screen.

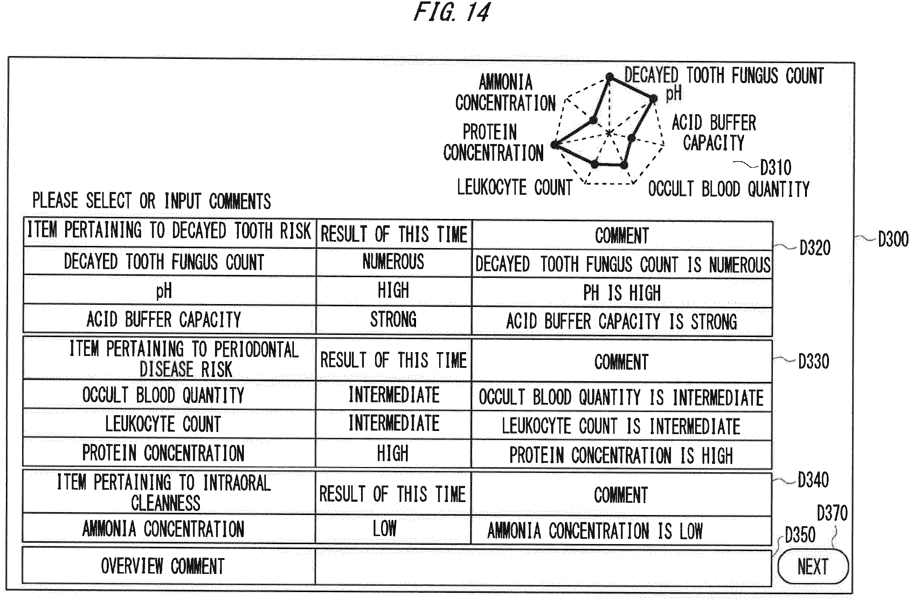

FIG. 14 is a diagram illustrating an example of the comment determination screen. As in FIG. 14, a comment determination screen D300 contains an examination result chart D310, a decayed tooth risk related field D320, a periodontal disease risk related field D330, an intraoral cleanness related field D340, an overview comment input field D350, and a "next" button D370.

The examination result chart D310 is similar to the chart D210 illustrated on the examination result screen D200 in step S104. The examination result chart D310 enables the user (the dentist) to visually recognize the measurement results of the examination of the examinee (the patient). The measurement results of the past examinations may also be displayed in the examination result chart D310. When selecting the comment, the user (the dentist) can select a more proper comment by referring to the past data.

The decayed tooth risk related field D320 contains an examination item field (of decayed tooth risk related items), an examination result field (of the results of this time) and a comment field. The "decayed tooth fungus count", the "pH" and the "acid buffer capacity" are described as the items related to the decayed tooth risk in the examination item field. The measurement results of the examination of this time with respect to the examination items are described in the examination result field. The comments about the examination results are described in the comment field. Of the comments acquired in step S105, one comment is described per examination item in the comment field. The comments described herein are temporary comments. The temporary comments are the comments described for the first time in, e.g., the comment table. The temporary comments may also be comments each exhibiting, e.g., a maximum selection count given in the past. A null value may remain in the comment field till a comment is selected or inputted. The temporary comments may also take null values in their fields. The same as the decayed tooth risk related field D320 is applied to the periodontal disease risk related field D330 and the intraoral cleanness related field D340.

The overview comment input field D350 is a field to which the user (the dentist) determines the measurement results and freely inputs the comments. The user (the dentist) inputs the comments, which are proper for being provided to the examinee (the patient) from the user (the dentist), to the overview comment input field D350. The user (the dentist) can input the comments of comprehensively determining the measurement results, intraoral diagnostic results, a lifestyle and other equivalent items toe the overview comment input field D350.

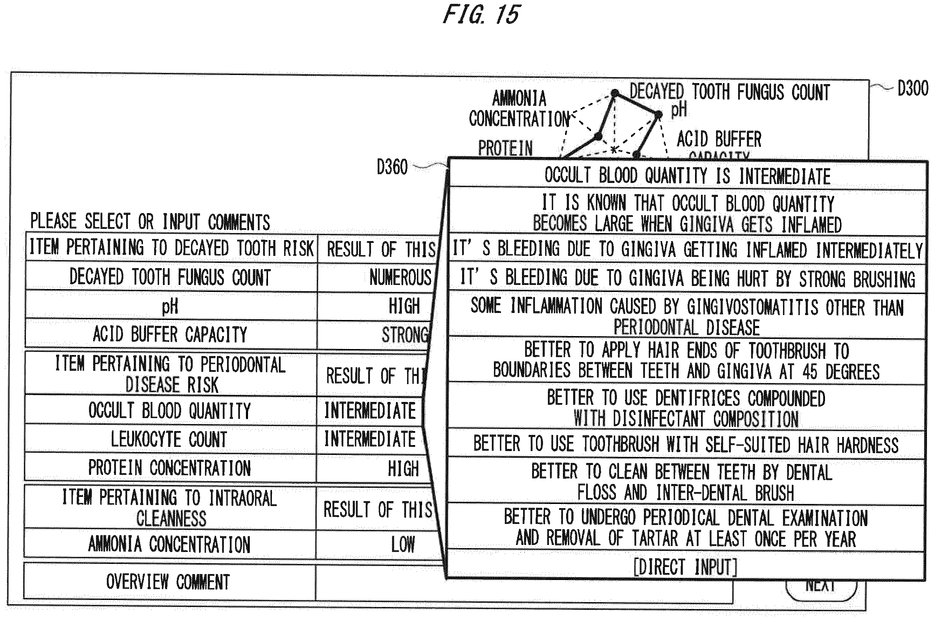

FIG. 15 is a diagram illustrating an example of another comment determination screen in the case of selecting the comment field of the "occult blood quantity" on the comment determination screen in FIG. 14. The measurement result of the "occult blood quantity" is "intermediate" in the examination of this time. Hence, as in FIG. 15, when the user (the dentist) selects the comment field of the "occult blood quantity", the control unit 11 displays a comment list D360 concerning the rank "intermediate" of the"occult blood quantity" on the display unit 14. Comments displayed in the comment list D360 are comments acquired from the comment table T220 of the comment DB in step S105. The user (the dentist) can select one or a plurality of comments from the comments displayed in the comment list D360. The comment list D360 contains a direct input field (character input field). In other words, the user (the dentist) can select the comment or the direct input field. When the user (the dentist) selects the direct input field, the control unit 11 displays, e.g., a cursor for inputting the characters on the display unit 14, and waits for the characters to be inputted from the input unit 13. The user (the dentist) is thereby enabled to freely directly input the comment about the rank "intermediate" of the "occult blood quantity" of the examinee (the patient) via the input unit 13 on this occasion. The selected comment or the directly inputted comment is displayed in the comment field with respect to the "occult blood quantity". Herein, the comments displayed in the comment field are the comments that are to be displayed in the examination result sheet. The same is applied to other examination items, in which the user (the dentist) selects or directly inputs the comments. The comment list D360 may include a null field as one of the comments.

Upon finishing selecting or inputting the comments with respect to the examination items and the overview comment, the comments concerning the respective examination items, which are selected or inputted by the user (the dentist), are displayed in the comment determination screen D300, and the overview comment inputted by the user (the dentist) is displayed in the overview comment input field. Null values may be given to the comment field and the overview comment field. Thereafter, when the user (the dentist) selects the "next" button D370, the processing advances to step S107. The user (the dentist) selects the "next" button D370, thereby determining the respective comments.

In step S107, the control unit 11 of the examination result sheet creation apparatus 10 stores all the comments (including the overview comment) determined in step S106 in the comment field of the examination of this time in the dental record table T100 of the examinee (the patient). For example, the comments selected or inputted with respect to the "occult blood quantity" are stored in the comment field of the "occult blood quantity" of the examination results given this time in the dental record table T100.

In step S108, the control unit 11 of the examination result sheet creation apparatus 10 adds, to the comment DB, the comment inputted directly by the user (the dentist) in the comments with respect to the respective examination items determined in step S106. For example, the user (the dentist) directly inputs the comment as the comment of the rank "intermediate" of the examination item "occult blood quantity", in which case the control unit 11 adds the directly inputted comment to the comment table T220 of the comment DB of the storage unit 12. The added comment is one of the comments displayed on the occasion of determining the comment on a next opportunity.

In step S109, the control unit 11 of the examination result sheet creation apparatus 10 displays a clinic name/dentist name input screen D400 on the display unit 14. The control unit 11 waits for the user (the dentist) to input a clinic name and a dentist name.

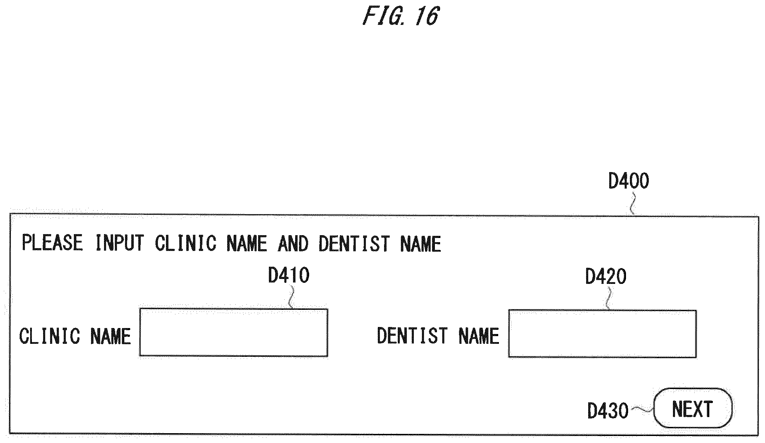

FIG. 16 is a diagram illustrating an example of the clinic name/dentist name input screen. The clinic name/dentist name input screen D400 contains a clinic name input field D410, a dentist name input field D420, and a "next" button D430.

The user (the dentist) inputs a name of the dental clinic and a name of the dentist that conduct the examination to the clinic name input field D410 and the dentist name input field D420 via the input unit 13. When the user (the dentist) selects the "next" button D430 after inputting the dentist name and other equivalent information, the control unit 11 stores the inputted clinic name and the inputted dentist name in the clinic name field and the dentist name field of the examination of this time in the dental record table T100 of the examinee (the patient). Through this process, it follows that the measurement results of the examination of this time, the comments about the measurement results, the name of the dental clinic and the name of the dentist that conduct the examination, are stored in the dental record table T100 of the examinee (the patient). When the clinic name and the dentist name are stored in the dental record table T100, the processing advances to step S110.

After performing the examination and determining the comments, the name of the dentist conducting the examination can be surely left as a record by causing the user to input the clinic name and the dentist name. The clinic name input field D410 and the dentist name input field D420 of the clinic name/dentist name input screen D400 are contained in the comment determination screen D300 in step S106, and may also be inputted together with the overview comment.

In step S110, the control unit 11 of the examination result sheet creation apparatus 10 creates the examination result sheet, and the print unit 15 prints the examination result sheet on the print paper. The printed examination result sheet is handed over to the examinee (the patient) from the user (the dentist).

The control unit 11 of the examination result sheet creation apparatus 10 may also display the examination result sheet on the display unit 14. At this time, the user (the dentist) can explain the examination results to the examinee (the patient) while seeing the examination result sheet displayed on the display unit 14 together with the examinee (the patient). Hereat, the control unit 11 of the examination result sheet creation apparatus 10 may not print the examination result sheet by the print unit 15. Electronic data of the examination result sheet may be stored on the storage unit 12.

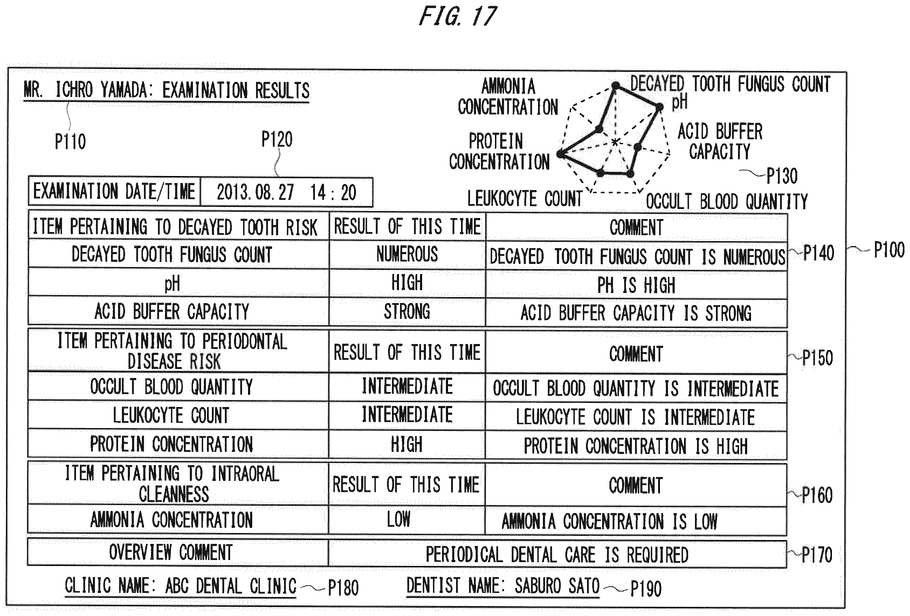

FIG. 17 is a diagram illustrating an example of the examination result sheet created by the examination result sheet creation apparatus. An examination result sheet P100 in FIG. 17 contains an examinee (patient) name P110, an examination date/time P120, a measurement result chart P130, a decayed tooth risk related field P140, a periodontal disease risk related field P150, an intraoral cleanness related field P160, an overview comment field P170, a clinic name field P180, and a dentist name field P190. A form of the examination result sheet is not limited to the examination result sheet P100 illustrated in FIG. 17.

The examinee (patient) name P110 is a name of the examinee (the patient) of the examination of this time. the name of the examinee (the patient) is extracted from the dental record table T100.

The examination date/time P120 is a date and time when performing the examination of this time. The date and the time when performing the examination of this time are extracted from the dental record table T100.

The measurement result chart P130 is a chart of the measurement results of the examination of this time. The measurement result chart P130 is the same as the chart D210 on the measurement result screen D200. The measurement results of the past examinations may be superposed on the measurement result chart P130. The superposition of the measurement results of the past examinations enables an easy comparison between the measurement results of the past examinations and the measurement results of the examination of this time.

The decayed tooth risk related field P140 contains descriptions of the "decayed tooth fungus count". The "pH" and the "acid buffer capacity" as the items pertaining to the caries risk, and also descriptions of the measurement results of the examination of this time and the comments about these items.

The comments are the comments determined in step S106. The same as the decayed tooth risk related field P140 is applied also to the periodontal disease risk related field P150 and the intraoral cleanness related field P160.

The overview comment field P170 contains a description of the comment inputted as the overview comment by the user (the dentist) in step S106.

The name of the dental clinic and the name of the dentist that conduct the examination are described in the clinic name field P180 and the dentist name field P190. The clinic name and the dentist name are the names inputted in step S110.

### (Edit of Comment DB)

In the operation flow described above, the user (the dentist) can input the comments for the respective examination items when the examination result sheet creation apparatus 10 creates the examination result sheet. However, when inputting the comments while creating the examination result sheet, the creation of the examination result sheet requires a considerable period of time as the case may be. Such being the case, there is herein made a description of how the comment table of the comment DB is edited previously (before creating the examination result sheet).

The control unit 11 of the examination result sheet creation apparatus 10 displays the examination items and the ranks, which become comment edit targets, on the display unit 14. The user (the dentist) selects the examination item and the rank (e.g., the "occult blood quantity" and "intermediate") desired to edit the comments from the examination items and the ranks displayed on the display unit 14. The control unit 11 of the examination result sheet creation apparatus 10 extracts, from the storage unit 12, the comment table containing the selected examination item and rank, and displays all the comments contained in this comment table. The user (the dentist) edits the comments such as adding, modifying and deleting the comments displayed on the display unit 14 via the input unit 13. When the user (the dentist) finishes editing the comments, the control unit 11 of the examination result sheet creation apparatus 10 stores the edited comments in the comment table. The post-editing comments stored in the comment table are used when creating the examination result sheet on the next opportunity.

The examination result sheet creation apparatus 10 enables the user (the dentist) to thus edit the comments contained in the comment DB. The user (the dentist) can add, modify and delete the comments described in the comment table of the comment DB before creating the examination result sheet. It is feasible to add a new comment and delete the comment not used (not selected) by editing the comment list. The comment DB can be edited, thereby enabling the user (the dentist) to quickly select a more proper comment when creating the examination result sheet.

### (Transmission to Comment Accumulation Server)

The examination result sheet creation apparatus 10 transmits the measurement results of the examination about the examination items of the examinee (the patient) and the comments made by the user (the dentist) with respect to the measurement results, to the comment accumulation server 30 defined as the external apparatus via the communication unit 16.

The dental record table T100 is stored with the examination items, the measurement results of the examination about the examination items, and the comments made by the user (the dentist) with respect to the measurement results by being associated with each other . Hence, the examination result sheet creation apparatus 10 may transmit the dental record table T100 of each examinee (the patient) to the comment accumulation server 30.

The comment accumulation server 30 analyzes the measurement results of the examination about the examination items of each examinee (the patient) and the comments made by the user (the dentist) with respect to the measurement results or the dental record table T100, which are transmitted from the examination result sheet creation apparatus 10, and is thereby enabled to acquire data of a comment usage frequency and other equivalent data items.

The examination result sheet creation apparatus 10 counts a number of comments used when creating the examination result sheet per examination item and per rank, and may transmit a comment count together with the comments to the comment accumulation server 30. The used comments may be counted on an attribute-by-attribute basis such as by gender and by age. When counted on the attribute-by-attribute basis, the examination result sheet creation apparatus 10 transmits attribute information together with the comments to the comment accumulation server 30.

The comment accumulation server 30 accumulates the comments per level of each examination item, which are inputted by the user (the dentist), and is thereby enabled to acquire the data of the comment usage frequency and other equivalent data items. The comment accumulation server 30 generates a new comment DB on the basis of the comment usage frequency, and can distribute the generated comment DB to each examination result sheet creation apparatus 10.

The examination result sheet creation apparatus 10 may store (transmit) the comments on the external apparatus like the external storage device in place of the transmission to the comment accumulation server 30. The external storage device is instanced by the USB memory connected to the examination result sheet creation apparatus 10 via the communication unit 16. The comments stored in the external apparatuses are accumulated and can be utilized.

### (Modified Example 1)

The example described above takes the configuration of directly inputting the clinic name and the dentist name; and, however, a modified example 1 is configured to make the clinic name and the dentist name selectable as on the comment determination screen in FIG. 15. Hereat, a clinic name table T300 and a dentist name table T400 are stored on the storage unit 12.

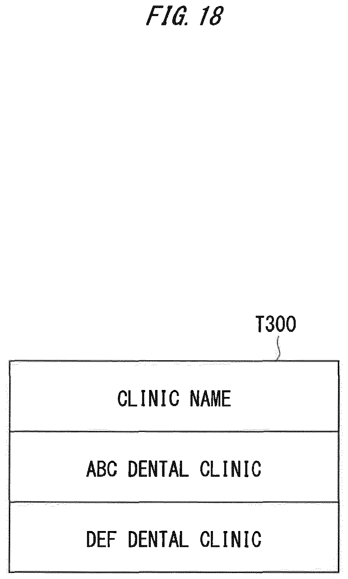

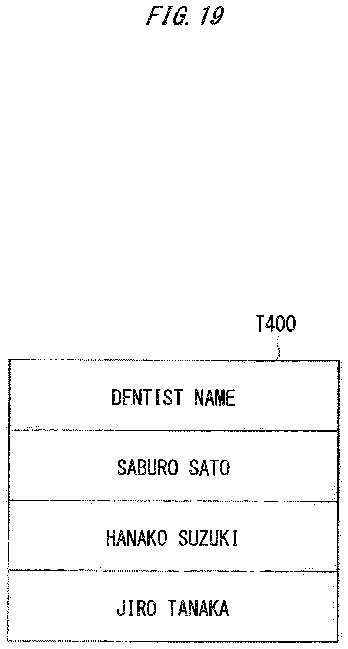

FIGS. 18 and 19 are diagrams illustrating the clinic name table and the dentist name table, respectively. The clinic name table T300 in FIG. 18 is stored with the names of the dental clinics to which the users (the dentists) using the examination result sheet creation apparatuses 10 belong. The dentist name table T400 in FIG. 19 is stored with the names of the users (the dentists) using the examination result sheet creation apparatuses 10.

When the clinic name and the dentist name are set selectable, the control unit 11 acquires, before displaying the clinic name/dentist name input screen D400, the clinic name and the dentist name respectively from the clinic name table T300 and the dentist name table T400 stored on the storage unit 12. The examination result sheet creation apparatus 10 displays the clinic name/dentist name input screen D400 on the display unit 14.

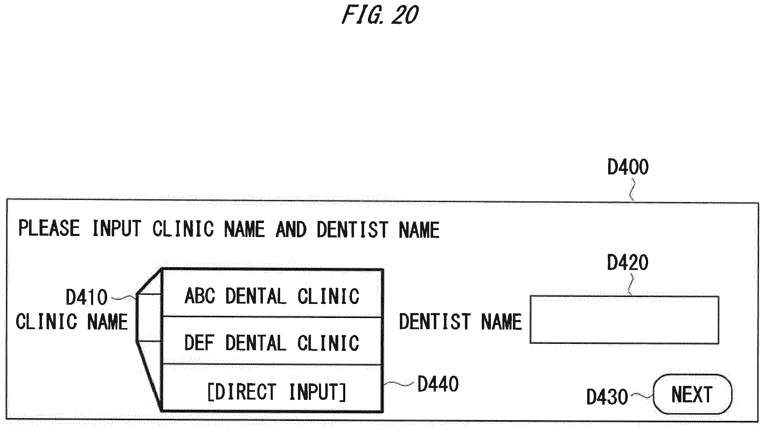

FIG. 20 is a diagram illustrating an example of the clinic name/dentist name input screen D400 when selecting the clinic name input field D410 in the case of making the clinic name and the dentist name selectable. As in FIG. 20, when the user (the dentist) selects the clinic name input field D410, the control unit 11 displays a clinic name list D440 on the display unit 14. The clinic names displayed in the clinic name list D440 are the clinic names acquired from the clinic name table T300 of the storage unit 12. The user (the dentist) can select one clinic name from the clinic names displayed in the clinic name list D440. The clinic name list D440 contains a direct input field (character input field). the user (the dentist) can select the clinic name or the direct input field from the clinic name list D440. When the user (the dentist) selects the direct input field, the control unit 11 displays the cursor for inputting the characters on the display unit 14 to enable the direct input of the name of the dental clinic to which the user (the dentist) belongs. The selected clinic name or the directly inputted clinic name is displayed in the clinic name input field D410. Herein, the clinic name displayed in the clinic name input field D410 becomes the inputted clinic name. Similarly, the user (the dentist) selects the dentist name or directly inputs the dentist name. The directly inputted clinic name and dentist name are stored in the clinic name table T300 and the dentist name table T400, respectively.

The clinic name table T300 and the dentist name table T400 can be edited similarly to the comment DB.

### (Modified Example 2)

In the example described above, the comment is acquired by selecting the "next" button D220 on the measurement result screen D200 in FIG. 10, and the comment determination screen D300 in FIG. 14 is displayed. Thereafter, the comment is determined by selecting the "next" button D370 on the comment determination screen D300, and the clinic name/dentist name input screen D400 in FIG. 16 is displayed. Further thereafter, the clinic name and the dentist name are determined by selecting the "next" button D430 on the clinic name/dentist name input screen D400, and the examination result sheet is printed.

In a modified example 2, the contents of the measurement result screen D200, the comment determination screen D300 and the clinic name/dentist name input screen D400 are displayed collectively on one screen. Hereat, when the contents of the respective screens do not fall within on one screen, the displays of the contents of the measurement result screen D200, the comment determination screen D300 and the clinic name/dentist name input screen D400 may be set mutually movable along a scroll bar displayed at an edge of the display unit 14.

### (Modified Example 3)

In the example described above, whenever the "next" button is pressed, the comments are stored in the dental record table T100. In a modified example 3, each time the comment is selected or inputted, the comment is stored in the dental record table T100 and the comment DB. Likewise, each time the clinic name and the dentist name are selected or inputted, the clinic name and the dentist name are stored in the dental record table T100, the clinic name table T300 and the dentist name table T400. At this time, the printing may be started by the "next" button.

According to the modified example 3, each time the comment is selected or inputted, the comment is stored in the storage unit 12, whereby the selected or inputted content is stored even when interrupting the selection or the input of the comment.

### (Modified Example 4)

In the above-mentioned, the examination result sheet creation apparatus 10 and the examination apparatus are configured separately in the examination result sheet creation system, and may also be configured integrally to operate as one examination apparatus so that the examination apparatus 20 performing the saliva examination includes the configuration of the examination result sheet creation apparatus 10.

### (Others)

Made herein is the description of how the examination result sheet using the examination results about the saliva of the examinee (the patient) is created; and, however, the embodiment 1 can be also applied on the occasion of creating the examination result sheet using the examination results by other methods.

### (Operation and Effect of Embodiment 1)

The examination result sheet creation apparatus 10 accepts the input of the patient information of the examinee (the patient) from the user (the dentist), and receives the measurement results of the saliva examination performed for the examinee (the patient) from the examination apparatus 20. The examination result sheet creation apparatus 10 stores the received measurement results in the dental record table T100 per examinee (per patient) in the storage unit 12. The examination result sheet creation apparatus 10 may also store, in the dental record table T100, the ranks given after ranking the measurement results per examination item as the measurement results. The examination result sheet creation apparatus 10 stores, on the storage unit 12, the comment DB containing the comments made per examination item and per rank and provided to the examinee (the patient). The comment DB contains the plurality of comments made per examination item and per rank. The examination result sheet creation apparatus 10 enables the user (the dentist) to select the comments contained in the comment DB or the comments directly inputted by the user (the dentist) as the comments provided with respect to the measurement results.

The examination result sheet creation apparatus 10 enables the user (the dentist) to select or input the comments proper for being provided to the examinee (the patient) by taking into consideration the condition and other equivalent states of the examinee (the patient), and to provide the selected or inputted comments to the examinee (the patient). The examination result sheet creation apparatus 10 saves the comment directly inputted by the user (the dentist) in the comment DB. The save comment is presented as one of the comments on the occasion of creating the examination result sheet afterward. The examination result sheet creation apparatus 10 enables the user (the dentist) to use the once-inputted comment without again directly inputting the comment by presenting the directly inputted comment on the occasion of creating the examination result sheet afterward.

The examination result sheet creation apparatus 10 makes it possible to provide the comments taking account of the results of the intraoral examination and the lifestyle of the individual examinee (individual patient) because of enabling the user (the dentist) to select the comments from the comment list and to directly input the comments when making the comments about the measurement results of each examination item. The examination result sheet creation apparatus 10 facilitates providing the unified comments for the examinee (the patient) in the same condition by reflecting the directly once-inputted comments in the options of the comments given afterward, and can prevent the evaluations of the intraoral state from being dispersed. The comments can be helpful for the user (the dentist) to assume every possibility about the intraoral state of the examinee (the patient).

The examination result sheet creation apparatus 10 enables the user (the dentist) to edit (e.g., add, modify and delete) the comments contained in the comment DB beforehand. The user (the dentist) can edit the comment DB beforehand and is thereby enabled to select the more proper comments more further quickly when creating the examination result sheet, and to provide the selected comments to the examinee (the patient).

### [Embodiment 2]

Next, an embodiment 2 will be described. The embodiment 2 has the common points to the embodiment 1. Accordingly, the discussion will focus mainly on different points, and the explanations of the common points to the embodiment 1 will be omitted.

### (Example of Configuration)

The examination result sheet creation system according to the embodiment 2 has the same configuration as the configuration of the examination result sheet creation system according to the embodiment 1. The embodiment 2 is different from the embodiment 1 in terms of such a point that the user (the dentist) determines the intraoral state, and makes comments about dental care methods and dental care products.

### (Operational Example)

An operational example of how the examination result sheet creation apparatus 10 according to the embodiment 2 creates the examination result sheet, will be described.

In the embodiment 2, similarly to the embodiment 1, the user (the dentist) of the examination result sheet creation apparatus 10 performs the saliva examination (examination) of the examinee (the patient), and thus creates the examination result sheet by using the examination apparatus 20.

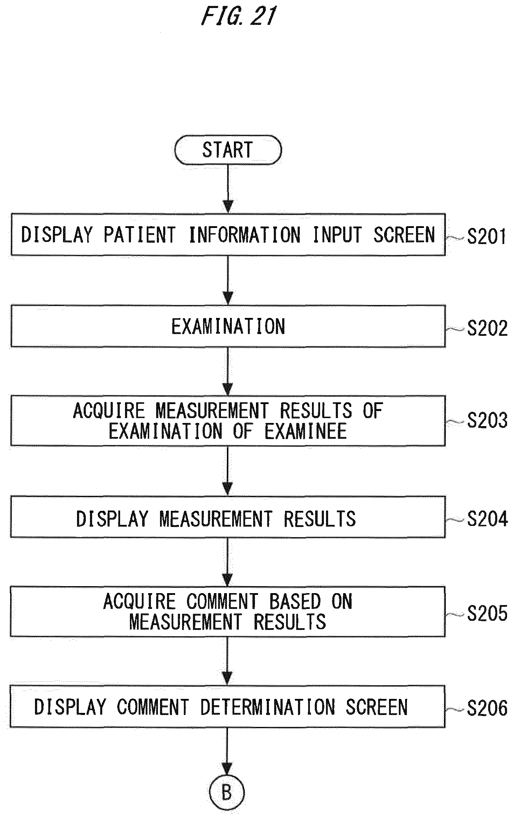

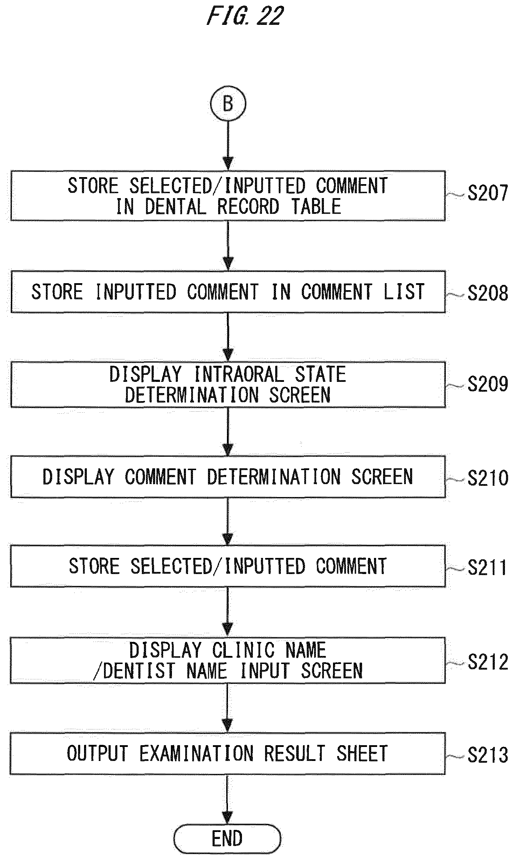

FIGS. 21 and 22 are flowcharts illustrating the operational example of creating the examination result sheet in the embodiment 2. The symbol "B" in FIG. 21 is continued to "B" in FIG. 22.

Operations in steps S201 through S208 are the same as the operations in steps S101 through S108 in the embodiment 1.

In step S209, the examination result sheet creation apparatus 10 displays an intraoral state determination screen on the display unit 14. The intraoral state determination screen is a screen containing a list of intraoral states. The user (the dentist) determines an intraoral state of the examinee (the patient) on the intraoral state determination screen, based on the saliva examination results and the intraoral state and other equivalent states of the examinee (the patient). Herein, the intraoral states are instanced by whether to have a tendency of the decayed tooth, a tendency of the periodontal disease and a tendency of the low intraoral cleanness.

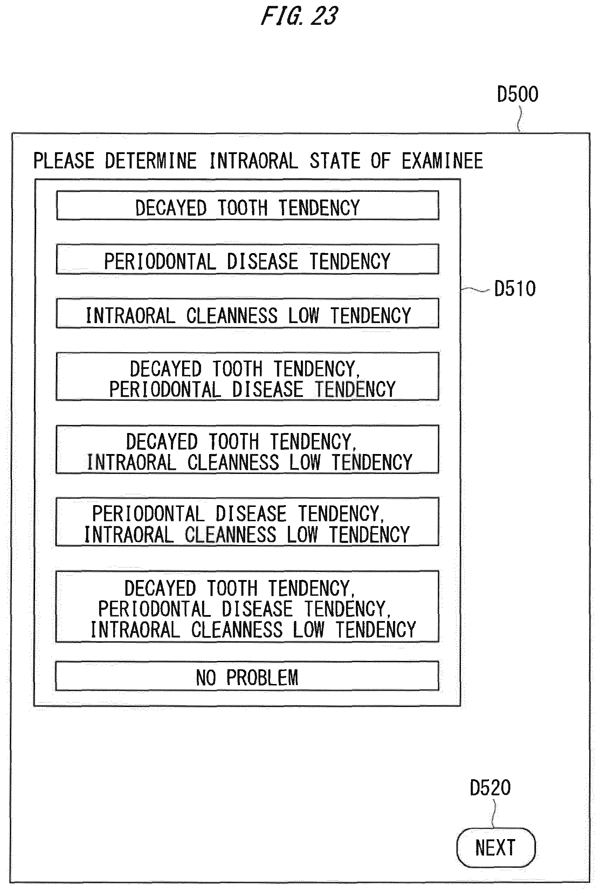

FIG. 23 is a diagram illustrating an example of the intraoral state determination screen. An intraoral state determination screen D500 in FIG. 23 contains an intraoral state list D510, and a "next" button D520.

The intraoral state list D510 contains 8 types of options, i.e., a "decayed tooth tendency", a "periodontal disease tendency", an "intraoral cleanness low tendency", a "decayed tooth tendency/periodontal disease tendency", a "decayed tooth tendency/intraoral cleanness low tendency", a "periodontal disease tendency/intraoral cleanness low tendency", a "decayed tooth tendency/periodontal disease tendency/intraoral cleanness low tendency", and a "no problem". The user (the dentist) selects one of the options of the intraoral state list D510 on the basis of the saliva examination results and the intraoral state of the examinee (the patient), thereby determining the intraoral state of the examinee (the patient). Thereafter, upon selecting the"next" button D520 by the user (the dentist), the processing advances to step S210. The user (the dentist) selects the"next" button D520, thereby settling the determination of the intraoral state.

In step S210, the control unit 11 acquires respective items of the dental care methods and product contributions of the dental care products from a contribution table in the storage unit 12 on the basis of the determination result of the intraoral state in step S209. The contributions are contributions for improving the intraoral state with respect to the respective items of the dental care methods and the respective products as the dental care products. The contribution table is previously stored on the storage unit 12. The contribution table contains the contributions for improving the intraoral states by being associated with the intraoral states with respect to the respective items of the dental care methods and the respective products as the dental care products. The contribution for improving the intraoral state is information provided to the examinee (the patient). The contributions are exemplified by those calculated by a statistical process from the accumulation data. For example, the contributions may be classified into ranks at an odds ratio calculated based on a logistic analysis in relation to whether the intraoral state is improved owing to each dental care method and each dental care product. The contributions are not limited to those described herein.

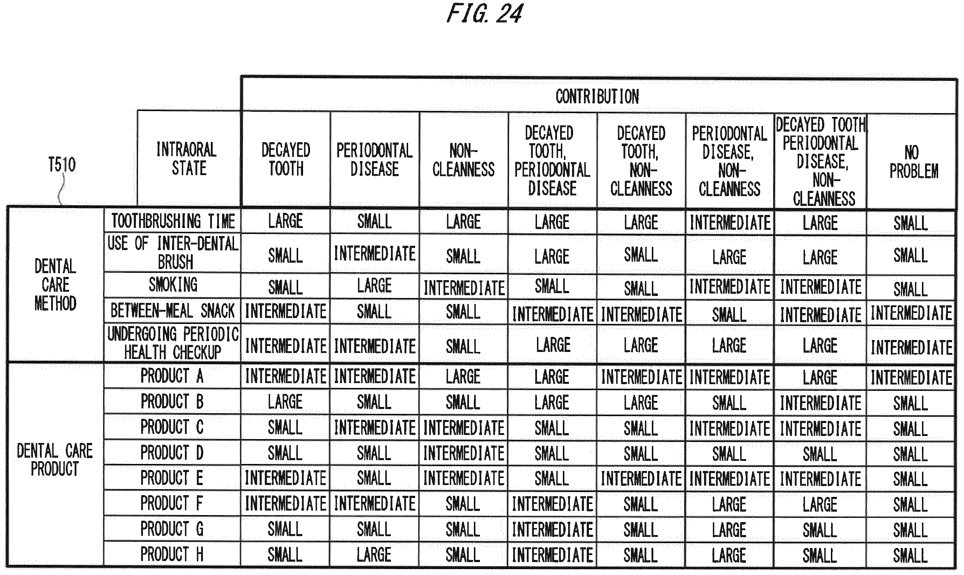

FIG. 24 is a diagram illustrating an example of the contribution table. In the contribution table T510 of the example of FIG. 24, the contributions for improving the intraoral states are ranked such as "large", "intermediate" and "small" per intraoral state with respect to the items of the dental care methods, i.e., 'toothbrushing time", "use of inter-dental brush, "smoking", "between-meal snack" and "undergoing periodic health checkup". In the contribution table T510, the contributions for improving the intraoral states are ranked such as "large", "intermediate" and "small" per intraoral state with respect to products A - H as the dental care products. Herein, in FIG. 24, "decayed tooth", "periodontal disease" and "non-clean" given in fields of the intraoral state indicate the "decayed tooth tendency", the "periodontal disease tendency" and the "intraoral cleanness low tendency" as the intraoral states. Further, the item "no problem" given in a field of the intraoral state indicates that there is no problem with respect to "decayed tooth", "periodontal disease" and "intraoral cleanness". The items of the dental care methods and the dental care products are not limited to those described above, but may be increased or decreased.

The control unit 11 acquires the comments from a dental care comment DB in the storage unit 12 per item of the dental care method and per product of the dental care products, based on the acquired contributions. The dental care comment DB is stored on the storage unit 12 beforehand. The dental care comment DB contains a comment table of comments about all the items of the dental care methods and all the dental care products. Each comment table contains a plurality of comments. The comment is a message provided to the examinee (the patient). The comment pertaining to the dental care method and the dental care product is also called a dental care comment.

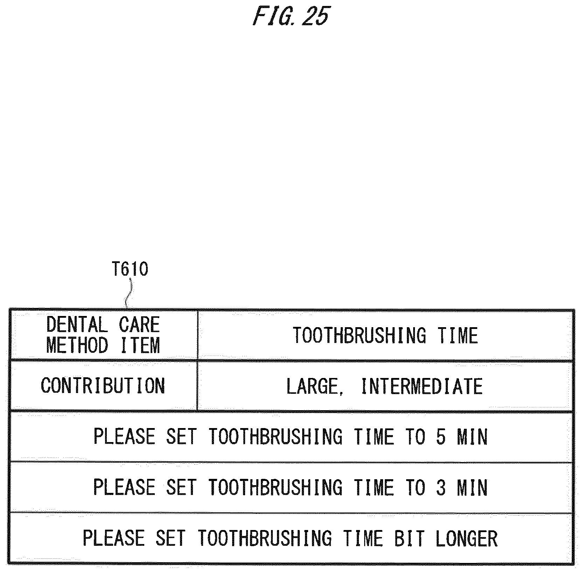

FIG. 25 is a diagram illustrating an example of the comment table containing the items, i.e., "toothbrushing time" and the contributions such as "large" or "intermediate" in the dental care methods of the dental care comment DB. A comment table T610 in FIG. 25 contains three comments with respect to the items, i.e., "toothbrushing time" and the contributions such as "large" or "intermediate" in the dental care methods. Herein, the comment of the contribution "large" and the comment of the contribution "intermediate" are commonized, but may also be separately provided.

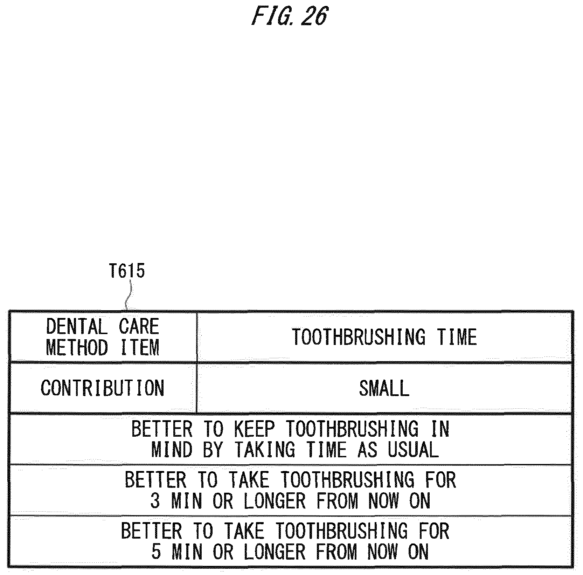

FIG. 26 is a diagram illustrating an example of the comment table containing the items, i.e., "toothbrushing time" and the contribution such as "small in the dental care methods of the dental care comment DB.

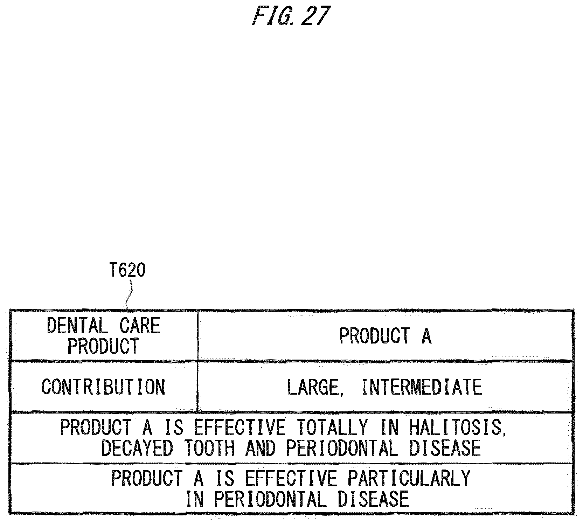

FIG. 27 is a diagram illustrating an example of the comment table of comments about "product A" as the dental care product and the contribution such as "large" or "intermediate" in the dental care comment DB. A comment table T620 in FIG. 27 contains two comments about "product A" as the dental care product.

The control unit 11 displays a dental care comment determination screen on the display unit 14. The dental care comment determination screen is a screen containing the items of the dental care methods, the products as the dental care products, the contributions for improving the intraoral states, and the comment field. The user (the dentist) determines the comments about the respective items of the dental care methods and the products as the dental care products on the dental care comment determination screen.

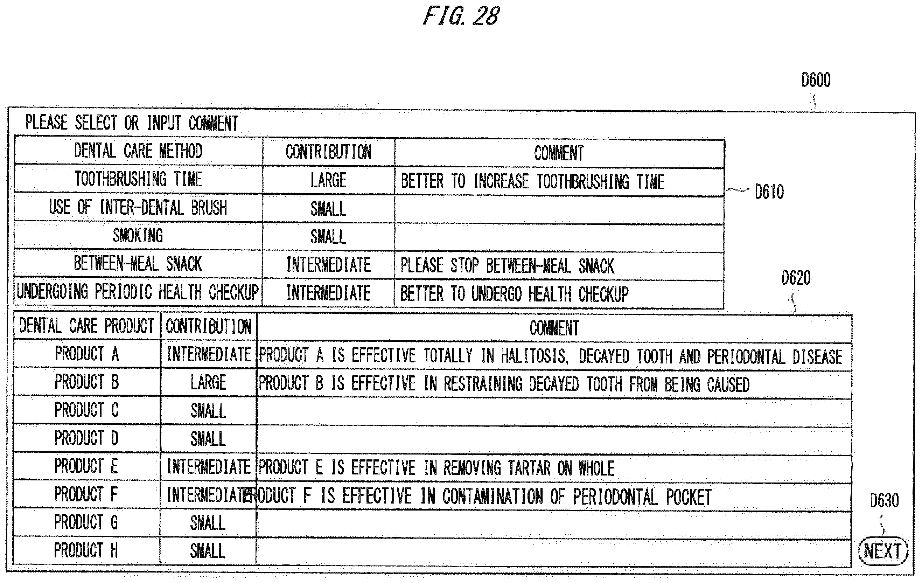

FIG. 28 is a diagram illustrating an example of the dental care comment determination screen. As in FIG. 28, a dental care comment determination screen D600 contains a field D610 pertaining to the dental care method, a field D620 pertaining to the dental care products, and a "next" button D630. Herein, the intraoral state is determined to be "decayed tooth tendency".

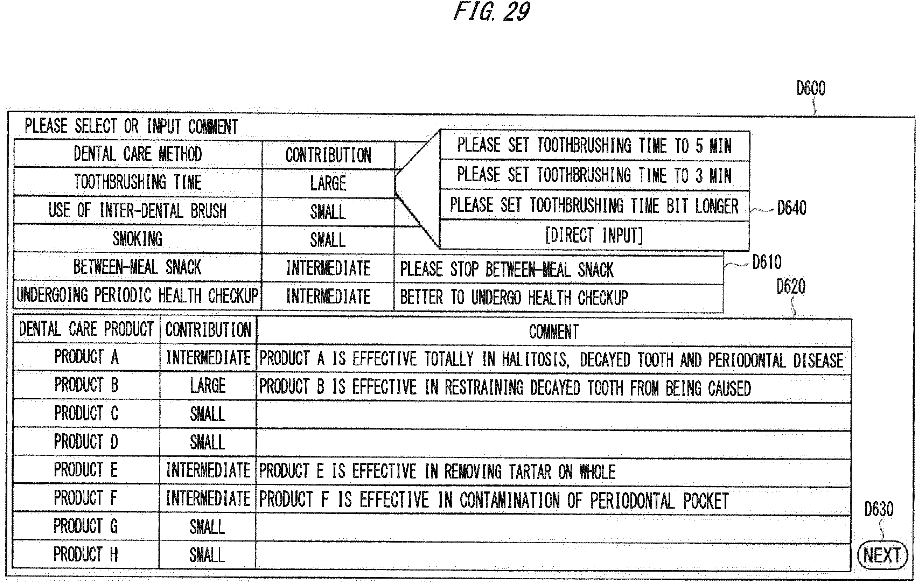

The field D610 pertaining to the dental care method contains an item field of the dental care method, a contribution field and a comment field. The items such as 'toothbrushing time", "use of inter-dental brush, "smoking", "between-meal snack" and "undergoing periodic health checkup" are described each as the dental care method in the item field of the dental care method. The contributions for improving the intraoral states with respect to the items of the dental care methods, are described in the contribution field. The comments about the items of the dental care methods are described in the comment field. The comment described in the comment field is one of the comments acquired from the dental care comment DB. The comment described herein is the temporary comment. The temporary comment is the comment described for the first time in, e.g., the comment table. The temporary comment may also be the comment exhibiting, e.g., the maximum selection count given in the past. A null value may remain in the comment field till the comment is selected or inputted. The temporary comment may also take a null value in its field. The same as the dental care method related field D610 is applied to the dental care product related field D620. FIG. 29 is a diagram illustrating an example of the dental care comment determination screen in the case of selecting the comment field of "toothbrushing time" on the dental care comment determination screen in FIG. 28. The contribution of "toothbrushing time" for improving the intraoral state is "large" in the determination of the intraoral state of this time. Hence, as in FIG. 29, when the user (the dentist) selects the comment field of "toothbrushing time", the control unit 11 displays the comment list D640 with respect to "toothbrushing time" on the display unit 14. The comments displayed in the comment list D640 are the comments acquired from the comment table T610 of the dental care comment DB. The user (the dentist) can select one or a plurality of comments from the comments displayed in the comment list D640. The comment list D640 contains the direct input field (character input field). In other words, the user (the dentist) can select the comment or the direct input field from the comment list D640. When the user (the dentist) selects the direct input field, the control unit 11 displays the cursor for inputting the characters on the display unit 14, and waits for the input unit 13 to input the characters. The user (the dentist) is thereby enabled to freely directly input the comment about "toothbrushing time" of the examinee (the patient) of this time through the input unit 13. The selected comment or the directly inputted comment is displayed in the comment field with respect to "toothbrushing time". Herein, the comments displayed in the comment field become the comments displayed in the examination result sheet. The user (the dentist) likewise selects the comment or directly inputs the comment also with respect to other examination items. The comment list D640 may include a null field as one of the comments.

When finishing selecting or inputting the comments about the items of the dental care methods and the dental care products, the comments selected or inputted by the user (the dentist) about the respective items and the respective products, are displayed in the dental care comment determination screen D600. A null value may also be given in the comment field. Thereafter, when the user (the dentist) selects the "next" button D630, the processing advances to step S211. The user (the dentist) selects the "next" button D630, thereby determining each comment.

In step S211, the control unit 11 stores all the comments determined in step S210 in the dental care field of the examination of this time in the dental care table T100 of the examinee (the patient). For example, the comment selected or inputted with respect to "toothbrushing time" is stored in the dental care comment field of "toothbrushing time" of the examination result of this time in the dental record table T100. In the embodiment 2, the dental record table T100 contains the item of the dental care method, the dental care product, the contribution and the dental care comment field.

The control unit 11 adds, to the comment DB, the comment directly inputted by the user (the dentist) in the comments about the respective examination items determined in step S210. For example, when the user (the dentist) directly inputs the comment as the comment of "toothbrushing time", the control unit 11 adds the directly inputted comment to the comment table T610 of the comment DB in the storage unit 12. The added comment is one of the comments displayed when determining the comment on the next opportunity.

### Step S212 is the same as step S109 in the embodiment 1.

In step S213, the control unit 11 creates the examination result sheet, and prints the examination result sheet on the print paper by the print unit 15. The printed examination result sheet is handed over to the examinee (the patient) from the user (the dentist).

The control unit 11 may display the examination result sheet on the display unit 14. Hereat, the user (the dentist) can explain the examination results to the examinee (the patient) while seeing the examination result sheet displayed on the display unit 14 together with the examinee (the patient). At this time, the control unit 11 of the examination result sheet creation apparatus 10 may not print the examination result sheet by the print unit 15. The electronic data representing the examination result sheet may also be stored on the storage unit 12.

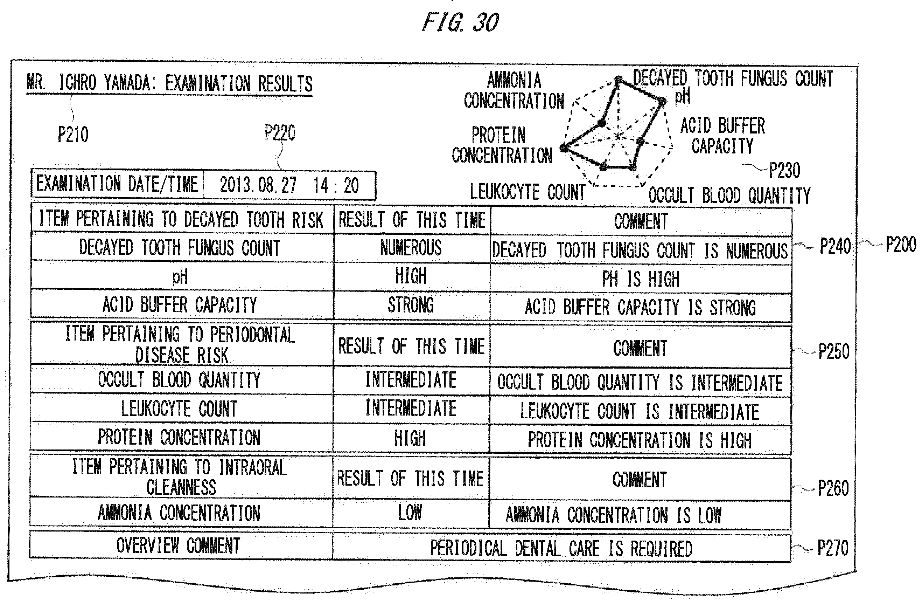

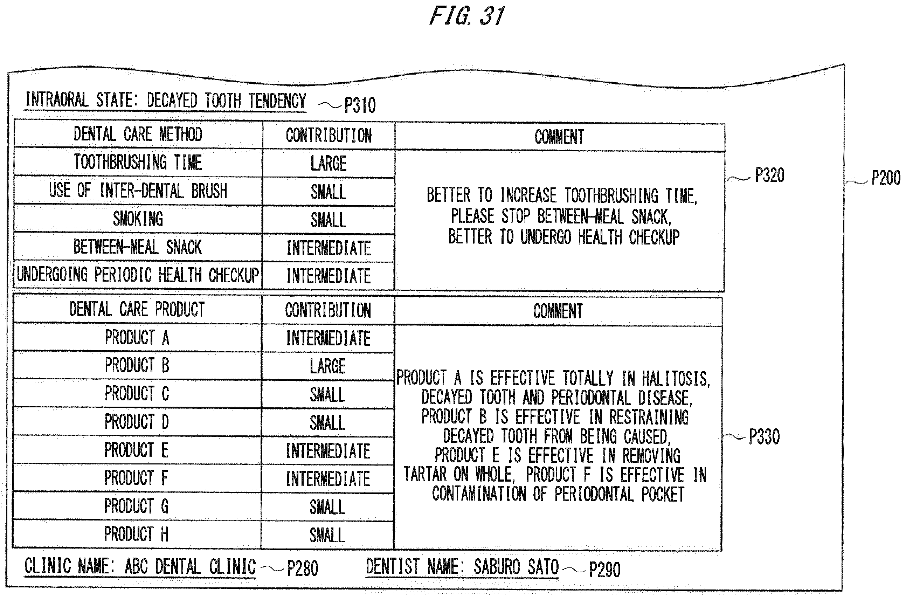

FIGS. 30 and 31 are diagrams illustrating examples of the examination result sheet created by the examination result sheet creation apparatus. A lower area of FIG. 30 is continued to an upper area of FIG. 31. A examination result sheet P200 in FIGS. 30 and 31 contains an examinee's (patient's) name P210, am examination date/time P220, a measurement result chart P230, a caries risk related field P240, a periodontal disease risk related field P250, an intraoral cleanness related field P260, an overview comment field P270, a clinic name field P280, and a dentist name field P290. The examination result sheet P200 further contains an intraoral state field P310, a dental care method related field P320, and a dental care product related field P330. A form of the examination result sheet is not limited to the examination result sheet P200 depicted in FIGS. 30 and 31.

The examinee's (patient's) name P210, the examination date/time P220, the measurement result chart P230, the caries risk related field P240, the periodontal disease risk related field P250, the intraoral cleanness related field P260, the overview comment field P270, the clinic name field P280 and the dentist name field P290 are the same as the corresponding fields of the examination result sheet P100 in the embodiment 1.

The intraoral state of the examinee (the patient), which is determined in step S209, is described in the intraoral state field P310.

The dental care method related items such as "toothbrushing time", "use of inter-dental brush, "smoking", "between-meal snack" and "undergoing periodic health checkup" are described in the dental care method related field P320, and the contributions for improving the intraoral states are described corresponding to these items. The comments selected or inputted in relation to the dental care methods are described in the comment field. The comments are the comments determined in step S210. The same as the dental care method related field P320 is also applied to the dental care product related field P330.

### (Others)

In the embodiment 2, the user (the dentist) makes the comments (dental care comments) about the dental care methods and the dental care products after determining the intraoral state of the examinee (the patient), and hence the comment table T210 may not contain the comments about the dental care methods and the dental care products.

### (Operation and Effect of Embodiment 2)

The examination result sheet creation apparatus 10 causes the user (the dentist) to determine the intraoral state. The examination result sheet creation apparatus 10 extracts the respective items of the dental care methods and the contributions, for improving the intraoral state, of the respective products as the dental care products on the basis of the determination result of the intraoral state. The examination result sheet creation apparatus 10 causes the user (the dentist) to select or input the comments with respect to the items of the dental care methods and the dental care products. The selected or inputted the comments are provided to the examinee (the patient).

The examinee (the patient) can effectively take care of the intraoral state such as the teeth by using the dental care method and the dental care product, which have the high contributions for improving the intraoral state, based on the description of the examination result sheet.

The embodiments discussed above can be carried out by being combined to the greatest possible degree.

### [Description of Reference Numerals and Symbols]

- 1: examination result sheet creation system
- 10: examination result sheet creation apparatus
- 11: control unit
- 12: storage unit
- 13: input unit
- 14: display unit
- 15: print unit
- 16: communication unit
- 20: examination apparatus
- 30: comment accumulation server
- 100: information processing apparatus
- 102: processor
- 104: memory
- 106: storage unit
- 108: input unit
- 110: output unit
- 112: communication control unit

## Claims

1. A system comprising an examination result sheet creation apparatus (10) and an external apparatus (30), the examination result sheet creation apparatus comprising:
a display unit (14) configured to display examination results of a saliva examination;
a control unit (11) configured to create an examination result sheet containing at least one examination result and a comment corresponding to the at least one examination result;
a storage unit (12) configured to store data used by the examination result sheet creation apparatus (10); and
a communication unit (16) configured to output information of the at least one examination result and a comment to the external apparatus (30),
**characterized in that**:
the display unit (14) is configured to display a plurality of comment candidates corresponding to the at least one examination result; and
the examination result sheet creation apparatus (10) comprises an input unit (13) configured to accept an input of a selection result of the plurality of comment candidates and configured to accept an input of a free descriptive comment,
wherein the control unit (11) is further configured to:
cause the display unit (14) to display an input field of a free descriptive comment about the examination result of the saliva examination, the free descriptive comment being inputted to the input field by using the input unit (13);
accept the free descriptive comment inputted to the input field;
store the at least one examination result and a comment selected from the comment candidates or the comment inputted as the free descriptive comment as being associated with each other in a comment database on the storage unit (12); and
store the free descriptive comment, if inputted, as one of the plurality of comment candidates corresponding to the at least one examination result on the storage unit (12);
wherein the comment in the examination result sheet comprises the comment selected from the comment candidates or the free descriptive comment, and
wherein the comment output by the communication unit (16) is the associated comment selected from the comment candidates or the associated comment inputted as the free descriptive comment to the external apparatus, and
wherein the external apparatus (30) is configured to:
accumulate the comments associated with each examination result;
determine a comment usage frequency for each examination result;
generate a new comment database on the basis of the comment usage frequency; and
distribute the new comment database to the examination result sheet creation apparatus (10).

2. The system according to claim 1, wherein the display unit (14) displays an edit screen of the comments about the at least one examination result of the saliva examination, and
the control unit (11) stores, on the storage unit (12), a result of executing an edit process corresponding to the plurality of comment candidates by using information inputted from the input unit (13).

3. The system according to any claim 1 or 2, wherein the control unit causes the display unit (14) to display a plurality of options about an intraoral state,
the input unit (13) accepts an input of a selection result of the option,
the control unit (11) causes the display unit (14) to display a plurality of dental care comment candidates corresponding to dental care methods and dental care products pertaining to the intraoral state corresponding to the selected option, and
the control unit (11) stores, on the storage unit (12), at least one dental care method or one dental care product and the dental care comment selected from the dental care comment candidates by being associated with each other, and creates the examination result sheet containing at least one dental care method or one dental care product and the dental care comment selected from the dental care comment candidates.

4. The system according to claim 3, wherein the control unit (11) causes the display unit (14) to display contributions for improving the intraoral state.

5. An examination result sheet creation method by which an examination result sheet creation apparatus (10) executes:
displaying examination results of a saliva examination and a plurality of comment candidates corresponding to the examination results and an input field of a free descriptive comment about the examination results;
accepting an input of a selection result of the plurality of comment candidates or the free descriptive comment inputted to the input field;
storing at least one examination result and the comment selected from the comment candidates or the free descriptive comment inputted to the input field as being associated with each other in a comment database on a storage unit (12),
storing the free descriptive comment, if inputted to the input field, as one of the plurality of comment candidates corresponding to the examination results on the storage unit (12),
creating an examination result sheet containing at least one examination result and the comment selected from the comment candidates or the free descriptive comment inputted to the input field; and
outputting information of the at least one examination result and the associated comment selected from the comment candidates or the associated free descriptive comment inputted to the input field, to an external apparatus (30),
and by which the external apparatus (30) executes:
accumulating the comments associated with each examination result;
determining a comment usage frequency for each examination result;
generating a new comment database on the basis of the comment usage frequency; and
distributing the new comment database to the examination result sheet creation apparatus.

6. The examination result sheet creation method according claim 5, wherein the examination result sheet creation apparatus (10) executes displaying an edit screen of the comments about the examination results of the saliva examination; and
storing, on the storage unit (12), a result of executing an edit process corresponding to the plurality of comment candidates by using information to be inputted.

7. The examination result sheet creation method according to claims 5 or 6, wherein the examination result sheet creation apparatus (10) executes displaying a plurality of options about an intraoral state;
accepting an input of a selection result of the option;
displaying a plurality of dental care comment candidates corresponding to dental care methods and dental care products pertaining to the intraoral state corresponding to the selected option; and
storing, on the storage unit, at least one dental care method or one dental care product and the dental care comment selected from the dental care comment candidates by being associated with each other, and creating the examination result sheet containing at least one dental care method or one dental care product and the dental care comment selected from the dental care comment candidates.

8. The examination result sheet creation method according to claim 7, wherein the examination result sheet creation apparatus executes displaying contributions for improving the intraoral state.

9. An examination apparatus comprising:
the system according to any one of claims 1 through 4; and
saliva examination means (20) to perform the saliva examination.

## Patentansprüche

1. System, das eine Einrichtung zum Erzeugen von Untersuchungsergebnisseiten (10) und eine externe Einrichtung (30) umfasst, wobei die Einrichtung zum Erzeugen von Untersuchungsergebnisseiten umfasst:
eine Anzeigeeinheit (14), die konfiguriert ist, um Untersuchungsergebnisse einer Speicheluntersuchung anzuzeigen;
eine Steuereinheit (11), die konfiguriert ist, um eine Untersuchungsergebnisseite zu erzeugen, die mindestens ein Untersuchungsergebnis und einen dem mindestens einen Untersuchungsergebnis entsprechenden Kommentar enthält;
eine Speichereinheit (12), die konfiguriert ist, um Daten zu speichern, die von der Einrichtung zum Erzeugen von Untersuchungsergebnisseiten (10) verwendet werden; und
eine Kommunikationseinheit (16), die konfiguriert ist, um Informationen über das mindestens eine Untersuchungsergebnis und einen Kommentar an die externe Einrichtung (30) auszugeben,
**dadurch gekennzeichnet, dass**:
die Anzeigeeinheit (14) konfiguriert ist, um eine Vielzahl von Kommentar-Kandidaten anzuzeigen, die dem mindestens einen Untersuchungsergebnis entsprechen; und
die Einrichtung zum Erzeugen von Untersuchungsergebnisseiten (10) eine Eingabeeinheit (13) umfasst, die konfiguriert ist, um eine Eingabe eines Auswahlergebnisses der Vielzahl von Kommentar-Kandidaten zu akzeptieren und konfiguriert ist, um eine Eingabe eines freien beschreibenden Kommentars zu akzeptieren,
wobei die Steuereinheit (11) weiter konfiguriert ist, um:
die Anzeigeeinheit (14) zu veranlassen, ein Eingabefeld eines freien beschreibenden Kommentars über das Untersuchungsergebnis der Speicheluntersuchung anzuzeigen, wobei der freie beschreibende Kommentar in das Eingabefeld unter Verwendung der Eingabeeinheit (13) eingegeben wird;
den in das Eingabefeld eingegebenen freien beschreibenden Kommentar zu akzeptieren;
das mindestens eine Untersuchungsergebnis und einen aus den Kommentar-Kandidaten ausgewählten Kommentar oder den als freien beschreibenden Kommentar eingegebenen Kommentar als einander zugeordnet in einer Kommentar-Datenbank auf der Speichereinheit (12) zu speichern; und
den freien beschreibenden Kommentar, falls eingegeben, als einen der Vielzahl von Kommentar-Kandidaten entsprechend dem mindestens einen Untersuchungsergebnis auf der Speichereinheit (12) zu speichern;
wobei der Kommentar in der Untersuchungsergebnisseite den aus den Kommentar-Kandidaten ausgewählten Kommentar oder den freien beschreibenden Kommentar umfasst, und
wobei der von der Kommunikationseinheit (16) ausgegebene Kommentar der aus den Kommentar-Kandidaten ausgewählte zugeordnete Kommentar oder der als freier beschreibender Kommentar in die externe Einrichtung eingegebene zugeordnete Kommentar ist, und
wobei die externe Einrichtung (30) konfiguriert ist, um:
die jedem Untersuchungsergebnis zugeordneten Kommentare zu akkumulieren;
eine Kommentarverwendungshäufigkeit für jedes Untersuchungsergebnis zu bestimmen;
eine neue Kommentar-Datenbank auf der Basis der Kommentarverwendungshäufigkeit zu generieren; und
die neue Kommentar-Datenbank an die Einrichtung zum Erzeugen von Untersuchungsergebnisseiten (10) zu verteilen.

2. System nach Anspruch 1, wobei die Anzeigeeinheit (14) einen Bearbeitungsbildschirm der Kommentare zu dem mindestens einen Untersuchungsergebnis der Speicheluntersuchung anzeigt, und
die Steuereinheit (11) auf der Speichereinheit (12) ein Ergebnis der Ausführung eines Bearbeitungsprozesses entsprechend der Vielzahl von Kommentar-Kandidaten unter Verwendung von Informationen, die von der Eingabeeinheit (13) eingegeben werden, speichert.

3. System nach einem der Ansprüche 1 oder 2, wobei die Steuereinheit die Anzeigeeinheit (14) veranlasst, eine Vielzahl von Optionen über einen intraoralen Zustand anzuzeigen,
die Eingabeeinheit (13) eine Eingabe eines Auswahlergebnisses der Option akzeptiert,
die Steuereinheit (11) die Anzeigeeinheit (14) veranlasst, eine Vielzahl von Zahnpflege-Kommentar-Kandidaten anzuzeigen, die Zahnpflegeverfahren und Zahnpflegeprodukten entsprechen, die sich auf den intraoralen Zustand beziehen, der der ausgewählten Option entspricht, und
die Steuereinheit (11) auf der Speichereinheit (12) mindestens ein Zahnpflegeverfahren oder ein Zahnpflegeprodukt und den aus den Zahnpflege-Kommentar-Kandidaten ausgewählten Zahnpflegekommentar speichert, indem sie einander zugeordnet werden, und die Untersuchungsergebnisseite erzeugt, die mindestens ein Zahnpflegeverfahren oder ein Zahnpflegeprodukt und den aus den Zahnpflege-Kommentar-Kandidaten ausgewählten Zahnpflegekommentar enthält.

4. System nach Anspruch 3, wobei die Steuereinheit (11) die Anzeigeeinheit (14) veranlasst, Beiträge zur Verbesserung des intraoralen Zustands anzuzeigen.

5. Verfahren zur Erzeugung von Untersuchungsergebnisseiten, bei dem eine Einrichtung zum Erzeugen von Untersuchungsergebnisseiten (10) Folgendes ausführt:
Anzeigen von Untersuchungsergebnissen einer Speicheluntersuchung und einer Vielzahl von Kommentar-Kandidaten, die den Untersuchungsergebnissen entsprechen, und eines Eingabefeldes für einen freien beschreibenden Kommentar zu den Untersuchungsergebnissen;
Akzeptieren einer Eingabe eines Auswahlergebnisses der Vielzahl von Kommentar-Kandidaten oder des freien beschreibenden Kommentars, der in das Eingabefeld eingegeben wurde;
Speichern mindestens eines Untersuchungsergebnisses und des aus den Kommentar-Kandidaten ausgewählten Kommentars oder des in das Eingabefeld eingegebenen freien beschreibenden Kommentars als einander zugeordnet in einer Kommentar-Datenbank auf einer Speichereinheit (12),
Speichern des freien beschreibenden Kommentars, falls er in das Eingabefeld eingegeben wurde, als einen der Vielzahl von Kommentar-Kandidaten, die den Untersuchungsergebnissen entsprechen, auf der Speichereinheit (12),
Erzeugen einer Untersuchungsergebnisseite, die mindestens ein Untersuchungsergebnis und den Kommentar enthält, der aus den Kommentar-Kandidaten oder dem freien beschreibenden Kommentar ausgewählt wurde, der in das Eingabefeld eingegeben wurde; und
Ausgeben von Informationen des mindestens einen Untersuchungsergebnisses und des zugeordneten Kommentars, der aus den Kommentar-Kandidaten ausgewählt wurde, oder des zugeordneten freien beschreibenden Kommentars, der in das Eingabefeld eingegeben wurde, an eine externe Einrichtung (30),
und bei dem die externe Einrichtung (30) Folgendes ausführt:
Akkumulieren der jedem Untersuchungsergebnis zugeordneten Kommentare;
Bestimmen einer Kommentarverwendungshäufigkeit für jedes Untersuchungsergebnis;
Generieren einer neuen Kommentardatenbank auf der Basis der Kommentarverwendungshäufigkeit; und
Verteilen der neuen Kommentardatenbank an die Einrichtung zum Erzeugen von Untersuchungsergebnisseiten.

6. Verfahren zum Erzeugen von Untersuchungsergebnisseiten nach Anspruch 5, wobei die Einrichtung zum Erzeugen von Untersuchungsergebnisseiten (10) das Anzeigen eines Bearbeitungsbildschirms der Kommentare zu den Untersuchungsergebnissen der Speicheluntersuchung; und
das Speichern, auf der Speichereinheit (12), eines Ergebnisses der Ausführung eines Bearbeitungsprozesses entsprechend der Vielzahl von Kommentar-Kandidaten unter Verwendung von einzugebenden Informationen ausführt.

7. Verfahren zum Erzeugen von Untersuchungsergebnisseiten nach Anspruch 5 oder 6, wobei die Einrichtung zum Erzeugen von Untersuchungsergebnisseiten (10) das Anzeigen einer Vielzahl von Optionen über einen intraoralen Zustand;
das Akzeptieren einer Eingabe eines Auswahlergebnisses der Option;
das Anzeigen einer Vielzahl von Zahnpflegekommentar-Kandidaten, die Zahnpflegemethoden und Zahnpflegeprodukten entsprechen, die sich auf den intraoralen Zustand beziehen, der der ausgewählten Option entspricht; und
das Speichern, auf der Speichereinheit, mindestens eines Zahnpflegeverfahrens oder eines Zahnpflegeprodukt und des aus den Zahnpflege-Kommentar-Kandidaten ausgewählten Zahnpflegekommentars ausführt, indem sie einander zugeordnet werden, und die Untersuchungsergebnisseite erzeugt, die mindestens ein Zahnpflegeverfahren oder ein Zahnpflegeprodukt und den aus den Zahnpflege-Kommentar-Kandidaten ausgewählten Zahnpflegekommentar enthält.

8. Verfahren zum Erzeugen von Untersuchungsergebnisseiten nach Anspruch 7, wobei die Einrichtung zum Erzeugen von Untersuchungsergebnisseiten Anzeigebeiträge zur Verbesserung des intraoralen Zustands ausführt.

9. Untersuchungseinrichtung, umfassend:
System nach einem der Ansprüche 1 bis 4; und
Speicheluntersuchungsmittel (20) zur Durchführung der Speicheluntersuchung.

## Revendications

1. Système comprenant un appareil de création de feuille de résultat d'examen (10) et un appareil externe (30), l'appareil de création de feuille de résultat d'examen comprenant:
une unité d'affichage (14) configurée pour afficher des résultats d'examen d'un examen salivaire ;
une unité de commande (11) configurée pour créer une feuille de résultat d'examen contenant au moins un résultat d'examen et un commentaire correspondant au au moins un résultat d'examen ;
une unité de stockage (12) configurée pour stocker des données utilisées par l'appareil de création de feuille de résultat d'examen (10) ; et
une unité de communication (16) configurée pour délivrer en sortie des informations concernant le au moins un résultat d'examen et un commentaire vers l'appareil externe (30),
**caractérisé en ce que** :
l'unité d'affichage (14) est configurée pour afficher une pluralité de commentaires candidats correspondant au au moins un résultat d'examen ; et
l'appareil de création de feuille de résultat d'examen (10) comprend une unité d'entrée (13) configurée pour accepter une entrée d'un résultat de sélection de la pluralité de commentaires candidats et configurée pour accepter une entrée d'un commentaire descriptif libre,
dans lequel l'unité de commande (11) est configurée en outre pour :
amener l'unité d'affichage (14) à afficher un champ d'entrée d'un commentaire descriptif libre concernant le résultat d'examen de l'examen salivaire, le commentaire descriptif libre étant entré dans le champ d'entrée en utilisant l'unité d'entrée (13) ;
accepter le commentaire descriptif libre entré dans le champ d'entrée ;
stocker le au moins un résultat d'examen et un commentaire sélectionné parmi les commentaires candidats ou le commentaire entré en tant que commentaire descriptif libre en étant associés l'un à l'autre dans une base de données de commentaires sur l'unité de stockage (12) ; et
stocker le commentaire descriptif libre, s'il est entré, en tant que l'un de la pluralité de commentaires candidats correspondant au au moins un résultat d'examen sur l'unité de stockage (12) ;
dans lequel le commentaire sur la feuille de résultat d'examen comprend le commentaire sélectionné parmi les commentaires candidats ou le commentaire descriptif libre, et
dans lequel le commentaire délivré en sortie par l'unité de communication (16) est le commentaire associé sélectionné parmi les commentaires candidats ou le commentaire associé entré en tant que commentaire descriptif libre dans l'appareil externe, et
dans lequel l'appareil externe (30) est configuré pour :
accumuler les commentaires associés à chaque résultat d'examen ;
déterminer une fréquence d'utilisation de commentaire pour chaque résultat d'examen ;
générer une nouvelle base de données de commentaires sur la base de la fréquence d'utilisation de commentaire ; et
distribuer la nouvelle base de données de commentaires à l'appareil de création de feuille de résultat d'examen (10).

2. Système selon la revendication 1, dans lequel l'unité d'affichage (14) affiche un écran d'édition des commentaires concernant le au moins un résultat d'examen de l'examen salivaire, et
l'unité de commande (11) stocke, sur l'unité de stockage (12), un résultat d'exécution d'un procédé d'édition correspondant à la pluralité de commentaires candidats en utilisant des informations entrées à partir de l'unité d'entrée (13).

3. Système selon une quelconque revendication 1 ou 2, dans lequel l'unité de commande amène l'unité d'affichage (14) à afficher une pluralité d'options concernant un état intrabuccal,
l'unité d'entrée (13) accepte une entrée d'un résultat de sélection de l'option, l'unité de commande (11) amène l'unité d'affichage (14) à afficher une pluralité de commentaires candidats de soin dentaire correspondant à des procédés de soin dentaire et des produits de soin dentaire se rapportant à l'état intrabuccal correspondant à l'option sélectionnée, et
l'unité de commande (11) stocke, sur l'unité de stockage (12), au moins un procédé de soin dentaire ou un produit de soin dentaire et le commentaire de soin dentaire sélectionné parmi les commentaires candidats de soin dentaire en étant associés l'un à l'autre, et crée la feuille de résultat d'examen contenant au moins un procédé de soin dentaire ou un produit de soin dentaire et le commentaire de soin dentaire sélectionné parmi les commentaires candidats de soin dentaire.

4. Système selon la revendication 3, dans lequel l'unité de commande (11) amène l'unité d'affichage (14) à afficher des contributions pour améliorer l'état intrabuccal.

5. Procédé de création de feuille de résultat d'examen par lequel un appareil de création de feuille de résultat d'examen (10) exécute :
un affichage de résultats d'examen d'un examen salivaire et d'une pluralité de commentaires candidats correspondant aux résultats d'examen et d'un champ d'entrée d'un commentaire descriptif libre concernant les résultats d'examen ;
une acceptation d'une entrée d'un résultat de sélection de la pluralité de commentaires candidats ou du commentaire descriptif libre entré dans le champ d'entrée ;
un stockage d'au moins un résultat d'examen et du commentaire sélectionné parmi les commentaires candidats ou le commentaire descriptif libre entré dans le champ d'entrée en étant associés l'un à l'autre dans une base de données de commentaires sur une unité de stockage (12),
un stockage du commentaire descriptif libre, s'il est entré dans le champ d'entrée, en tant que l'un de la pluralité de commentaires candidats correspondant aux résultats d'examen sur l'unité de stockage (12),
une création d'une feuille de résultat d'examen contenant au moins un résultat d'examen et le commentaire sélectionné parmi les commentaires candidats ou le commentaire descriptif libre entré dans le champ d'entrée ; et
une délivrance en sortie d'informations concernant le au moins un résultat d'examen et le commentaire associé sélectionné parmi les commentaires candidats ou le commentaire descriptif libre associé entré dans le champ d'entrée, vers un appareil externe (30),
et par lequel l'appareil externe (30) exécute :
une accumulation des commentaires associés à chaque résultat d'examen ;
une détermination d'une fréquence d'utilisation de commentaire pour chaque résultat d'examen ;
une génération d'une nouvelle base de données de commentaires sur la base de la fréquence d'utilisation de commentaire ; et
une distribution de la nouvelle base de données de commentaires à l'appareil de création de feuille de résultat d'examen.

6. Procédé de création de feuille de résultat d'examen selon la revendication 5, dans lequel l'appareil de création de feuille de résultat d'examen (10) exécute un affichage d'un écran d'édition des commentaires concernant les résultats d'examen de l'examen salivaire ; et
un stockage, sur l'unité de stockage (12), d'un résultat d'exécution d'un procédé de modification correspondant à la pluralité de commentaires candidats en utilisant des informations à entrer.

7. Procédé de création de feuille de résultat d'examen selon les revendications 5 ou 6, dans lequel l'appareil de création de feuille de résultat d'examen (10) exécute un affichage d'une pluralité d'options concernant un état intrabuccal ;
une acceptation d'une entrée d'un résultat de sélection de l'option ;
un affichage d'une pluralité de commentaires candidats de soin dentaire correspondant à des procédés de soin dentaire et à des produits de soin dentaire se rapportant à l'état intrabuccal correspondant à l'option sélectionnée ; et
un stockage, sur l'unité de stockage, d'au moins un procédé de soin dentaire ou d'un produit de soin dentaire et du commentaire de soin dentaire sélectionné parmi les commentaires candidats de soin dentaire en étant associés l'un à l'autre, et une création de la feuille de résultat d'examen contenant au moins un procédé de soin dentaire ou un produit de soin dentaire et le commentaire de soin dentaire sélectionné parmi les commentaires candidats de soin dentaire.

8. Procédé de création de feuille de résultat d'examen selon la revendication 7, dans lequel l'appareil de création de feuille de résultat d'examen exécute un affichage de contributions pour améliorer l'état intrabuccal.

9. Appareil d'examen comprenant :
le système selon l'une quelconque des revendications 1 à 4 ; et
des moyens d'examen salivaire (20) pour mettre en œuvre l'examen salivaire.
